# FASCICULE DE BREVET EUROPEEN

(11) **EP 1 931 320 B1**
(45) Date de publication et mention de la délivrance du brevet: **15.06.2022**
(21) Numéro de dépôt: 06831231.3
(22) Date de dépôt: 27.09.2006
(51) Int. Cl.: A61K 9/50, A61K 9/20, A61K 9/48, A61K 31/155, A61K 31/522

(54) **MICROPARTICULES ENROBÉES A LIBERATION MODIFIEE D'AU MOINS UN PRINCIPE ACTIF ET FORME GALENIQUE ORALE LES COMPRENANT**
MIKROPARTIKEL MIT MODIFIZIERTER FREISETZUNG VON MINDESTENS EINEM AKTIVEN PRINZIP UND ORALE DARREICHUNGSFORM DAVON
MICROPARTICLES WITH MODIFIED RELEASE OF AT LEAST ONE ACTIVE PRINCIPLE AND ORAL DOSAGE FORM COMPRISING THE SAME

(30) Priorité: 30.09.2005 FR 0552985
(43) Date de publication de la demande: 18.06.2008
(73) Titulaire: Flamel Ireland Limited, Dublin 15 (IE)
(72) Inventeur: DARGELAS, Frédéric, F-33600 Pessac (FR); GUIMBERTEAU, Florence, F-33450 Montussan (FR); CASTAN, Catherine, F-69510 Soucieu en Jarrest (FR); MEYRUEIX, Rémi, F-69009 Lyon (FR); SOULA, Gérard, F-69330 Meyzieu (FR)
(74) Mandataire: Plasseraud IP
(86) Numéro de dépôt international: PCT/FR2006/050944
(87) Numéro de publication internationale: WO 2007/036671

(56) Documents cités:
- WO-A-2005/007138
- US-B1- 6 228 398

## Description

### DOMAINE DE L'INVENTION

Le domaine de la présente invention est celui des systèmes microparticulaires à libération modifiée, par exemple retardée, prolongée et/ou pulsée, de principe(s) actif(s) (PA, désignant un ou plusieurs principes actifs), plus spécialement destinés à une administration par voie orale.

Les PA envisagés dans la présente invention sont notamment des PA pharmaceutiques, et en particulier ceux dont l'absorption se fait principalement dans le petit intestin. L'invention vise en premier lieu les microparticules enrobées élémentaires qui régissent chacune, de manière indépendante mais globalement homogène, la libération in vivo modifiée, à savoir notamment retardée et prolongée, du PA qu'elles renferment.

Prises ensemble, ces microparticules peuvent entrer dans la constitution de formes galéniques sèches telles que des comprimés, des sachets de poudre, des poudres pour suspension à reconstituer ou des gélules ou de formes galéniques liquides telles que des sirops ou des suspensions.

### CONTEXTE DE L'INVENTION

L'un des buts essentiels des formes pharmaceutiques à libération modifiée, que ce soit des formes à libération prolongée ou bien des formes à libération retardée ou à libération pulsée ou des combinaisons de ces formes à libération modifiée avec des formes à libération immédiate, est de prolonger la durée d'action pendant laquelle la concentration plasmatique en PA est supérieure au niveau minimal d'efficacité thérapeutique tout en maintenant la biodisponibilité du PA au niveau le plus élevé possible.

Un deuxième but essentiel des formes pharmaceutiques à libération modifiée est de garantir que le principe actif sera effectivement libéré. Ce point est particulièrement important dans le cas des anti-diabétiques ou des anti-hypertenseurs pour lesquels la bio-absorption du PA est vitale pour le patient.

Le profil de concentration plasmatique d'un PA comprend généralement une première phase pendant laquelle la concentration croît jusqu'à son maximum. Elle est suivie d'une deuxième phase, au cours de laquelle la concentration diminue par exemple de façon mono- ou bi-exponentielle.

La première phase correspond généralement à la phase de bio-absorption du principe actif. La deuxième phase correspond à la phase de distribution et d'élimination du principe actif. Les constantes cinétiques décrivant la deuxième phase de distribution et d'élimination sont fixées par la nature du principe actif.

Afin de prolonger la durée d'action du PA, on peut donc influer essentiellement sur la première phase, c'est-à-dire sur la bio-absorption du principe actif. Deux stratégies non exclusives l'une de l'autre sont envisageables.

La première stratégie consiste à augmenter la durée de bio-absorption du PA, de façon à allonger la phase d'absorption et ainsi, à prolonger la durée pendant laquelle la concentration plasmatique est maximale. Concrètement, cela revient à étaler le profil de concentration plasmatique sur une durée accrue.

La deuxième stratégie consiste à retarder le pic de concentration plasmatique tout en le maintenant le plus élevé possible. De cette façon, la phase de décroissance (distribution/élimination du PA) survient le plus tard possible et la durée d'action du PA est ainsi accrue, particulièrement lorsque la forme à libération modifiée est utilisée en conjonction avec une forme à libération immédiate qui assure la couverture thérapeutique dans les premiers instants après l'administration.

Dans les deux stratégies, il convient de maintenir la biodisponibilité du PA à un niveau élevé. Celle-ci peut être évaluée en mesurant l'aire sous la courbe de la concentration plasmatique du PA en fonction du temps.

Afin de prolonger la durée d'action d'un PA, les formes à libération prolongée libèrent lentement et de façon continue le PA, sur une durée de plusieurs heures, par exemple 8 heures. Cette libération lente et continue permet au PA d'être absorbé sur une durée prolongée, à condition que le PA séjourne devant sa fenêtre de bio-absorption pendant une durée suffisante. Cependant, de nombreux PA ont une fenêtre de bio-absorption étroite et localisée dans les parties hautes du petit intestin (duodénum, jéjunum). Dans ce cas, le temps de séjour du PA devant sa fenêtre de bio-absorption est limité, si bien que pour de telles formes à libération prolongée, une grande partie du PA est libérée hors de la fenêtre de bio-absorption et n'est pas absorbée. La concentration plasmatique en PA reste faible (faible bio-disponibilité) et la durée d'action n'est pas augmentée.

Un autre type de forme à libération modifiée est constitué par les formes à libération retardée entériques. Le PA n'est pas libéré tant que la forme reste dans l'estomac à pH acide. En revanche, la libération est rapide dès que le pH s'élève, généralement lorsque la forme pénètre dans le petit intestin. De telles formes pharmaceutiques entériques sont dites "pH-dépendantes". Le PA est rapidement libéré devant sa fenêtre de bio-absorption. La biodisponibilité peut alors être élevée, mais au détriment de la durée de bio-absorption. En outre, le pic de concentration plasmatique est précoce, particulièrement lorsque la vidange gastrique est rapide, ce qui est le cas à jeun. Il s'ensuit que de telles formes pharmaceutiques ont une durée d'action limitée.

Un deuxième inconvénient des formes entériques provient de la grande variabilité de la durée de la vidange gastrique chez un même individu et entre deux individus différents. Cela conduit à une très grande variabilité inter- et intra-individuelle des profils de concentration plasmatique du PA. Cette variabilité n'est pas acceptable pour des PA tels que les anti-hypertenseurs ou les anti-diabétiques, car elle met en danger les patients. Pour ces patients il est en effet vital que le PA soit effectivement libéré au moment voulu, même en cas de vidange gastrique anormalement tardive.

Le brevet US 6,228,398 divulgue des compositions multiparticulaires de principe actif, comprenant un composant à libération modifiée et un composant à libération immédiate. La demande de brevet WO2005/007138 décrit des formulations pharmaceutiques à libération contrôlée de principe actif, qui peuvent être à base de systèmes microparticulaires.

Pour pallier ces insuffisances, il a été proposé dans la demande de brevet WO-A-03/030878, une forme à libération modifiée du PA selon un double mécanisme pH-dépendant et temps-dépendant. La libération temps-dépendante est déclenchée au bout d'une durée prédéterminée de séjour dans l'estomac. La libération pH-dépendante intervient sous l'effet d'une remontée du pH, lorsque la forme pharmaceutique passe de l'estomac à l'intestin.

Ces deux facteurs déclencheurs de libération mis en parallèle confèrent au système galénique une grande sécurité d'emploi. La libération prolongée du PA est ainsi garantie après un temps de latence préréglé, même si la variation de pH n'est pas intervenue comme déclencheur, c'est à dire même si la forme galénique n'est pas sortie de l'estomac pour entrer dans l'intestin. Cette forme pharmaceutique représente un progrès considérable sur les formes entériques car elle garantit que le PA sera libéré, même en cas de rétention gastrique anormalement longue.

Cette forme selon WO-A-03/030878 est cependant perfectible car afin d'augmenter la durée d'action, il serait très utile de pouvoir étendre la durée de bio-absorption. A cette fin, il conviendrait de disposer d'une forme pharmaceutique à double mécanisme de libération (pH et temps) pour laquelle a) le temps de latence dans l'estomac, b) le pH de déclenchement de la libération dans le petit intestin et c) la vitesse de libération du PA dans l'intestin, soient ajustables en fonction de la nature du PA, de l'étendue de sa fenêtre de bio-absorption et de ses conditions d'administration.

Si on considère par exemple le cas d'un PA administré à l'état nourri et ayant une fenêtre de bio-absorption étroite, la vidange gastrique sera prolongée, mais le temps de passage devant la fenêtre de bio-absorption sera relativement court. Il conviendrait donc que le temps de latence dans l'estomac soit relativement long, que la libération survienne dès l'entrée de la forme pharmaceutique dans l'intestin et enfin que cette libération soit assez rapide afin de libérer le PA dans sa fenêtre de bio-absorption.

A l'inverse, dans le cas d'un PA administré à jeun et qui présente une fenêtre de bio-absorption étendue sur tout le petit intestin, la vidange gastrique sera rapide mais le temps de passage devant la fenêtre de bio-absorption sera relativement long. Il conviendrait donc que le temps de latence dans l'estomac soit court et que la libération du PA soit déclenchée assez tardivement dans l'intestin et/ou soit prolongée.

Il y a donc intérêt à disposer d'une forme pharmaceutique à double mécanisme de libération (pH et temps) pour laquelle il est possible d'ajuster commodément et indépendamment les uns des autres les trois paramètres suivants :
a) le temps de latence dans l'estomac,
b) le pH de déclenchement de la libération du PA dans l'intestin,
c) la vitesse de libération du PA dans l'estomac et/ou dans l'intestin.

Cela permettrait notamment d'ajuster au mieux la délivrance du PA en fonction de sa fenêtre d'absorption avec pour conséquences d'optimiser l'absorption et donc la durée d'action du PA, de limiter les effets secondaires et dans certains cas les doses et d'améliorer le confort des patients et l'observance du traitement en limitant le nombre de prises.

### OBJECTIFS DE L'INVENTION

Dans un tel état de la technique, l'un des objectifs essentiels de la présente invention est de fournir un nouveau système galénique multimicroparticulaire pour l'administration orale de PA absorbés dans le tractus gastro-intestinal au moins au niveau des parties hautes du tractus gastro-intestinal, qui permette d'augmenter le temps pendant lequel la concentration plasmatique en PA est supérieure ou égale à la concentration plasmatique minimale d'efficacité thérapeutique.

Un autre objectif de la présente invention est de proposer un système qui assure la libération modifiée du PA, grâce à un double mécanisme de libération "temps dépendant" et "pH dépendant". Ces deux facteurs déclencheurs de la libération de PA mis en parallèle, garantissent la libération du PA après un temps de latence préréglé, et ce même si la variation du pH n'est pas intervenue comme déclencheur.

Un autre objectif de la présente invention est de fournir un nouveau système galénique multi-microparticulaire pour l'administration de PA qui permette d'ajuster indépendamment les uns des autres les trois paramètres suivants :
a) le temps de latence précédant la libération du PA dans l'estomac, y compris jusqu'à supprimer ce temps de latence,
b) le pH de déclenchement de la libération du PA dans l'intestin,
c) la vitesse de libération du PA dans l'estomac et/ou dans l'intestin.

Un autre objectif essentiel de la présente invention est de fournir un système galénique multimicroparticulaire qui garantit la libération du PA en dépit de la variabilité inter- et intra-individuelle de la vidange gastrique.

Un autre objectif de la présente invention est de proposer une forme galénique formée au moins en partie d'une pluralité de microparticules enrobées évitant l'emploi de fortes quantités d'enrobant.

Un autre objectif de la présente invention est de proposer une forme pharmaceutique comprenant une pluralité de microparticules enrobées permettant de présenter le PA sous une forme facile à avaler : sachet, suspension ou comprimé orodispersible par exemple.

Un autre objectif de l'invention est de proposer une forme pharmaceutique formée au moins en partie d'une pluralité de microparticules enrobées permettant de mélanger plusieurs PA différents.

Un autre objectif de la présente invention est de proposer une forme pharmaceutique formée au moins en partie d'une pluralité de microparticules enrobées présentant différents temps de latence et/ou différentes vitesses de libération.

### BREVE DESCRIPTION DE L'INVENTION

S'étant fixés les objectifs ci-dessus, parmi d'autres, les inventeurs ont eu le mérite de mettre au point, une forme galénique à libération retardée et prolongée, multimicroparticulaire, à libération "temps dépendant" et à libération "pH dépendante", à temps de latence et temps de libération réglables indépendamment l'un de l'autre et individuellement recouvertes par au moins deux pellicules d'enrobage permettant la libération retardée et prolongée du PA.

Les inventeurs ont également eu le mérite de réfléchir à une stratégie adaptée pour atteindre les objectifs qu'ils se sont assignés. Cette stratégie est la suivante :
1. Il convient de ne pas libérer dans l'estomac de façon précoce une quantité trop importante de PA ou l'essentiel du PA, afin de pouvoir prolonger le temps de bioabsorption ;
2. Il convient de pouvoir régler le pH de déclenchement de la libération du PA dans l'intestin ;
3. Il convient de libérer le PA dans l'intestin, progressivement et à vitesse réglable.

Ainsi, l'invention concerne tout d'abord des microparticules "réservoir" enrobées contenant au moins un principe actif (PA) et étant du type de celles :
- constituées par des particules de PA recouvertes chacune d'au moins deux pellicules d'enrobage distinctes (i.e. de composition différente),
- de diamètre moyen inférieur à 2000 microns et de préférence compris entre 50 et 800 microns et, plus préférentiellement encore, entre 100 et 600 microns ;
caractérisées en ce que, en combinaison, ces pellicules d'enrobage sont aptes :
- à assurer une libération du PA régie par deux mécanismes distincts de déclenchement, l'un basé sur une variation de pH et l'autre permettant la libération du PA au bout d'un temps prédéterminé de résidence dans l'estomac,
- à induire un comportement de dissolution in vitro (réalisé dans un dissolutest à palettes conforme à la Pharmacopée européenne 5.2 ou appareil de type II dans l'US Pharmacopeia 28-NF 23, maintenu à 37°C et agité à 100 tours/min) tel que :
   - à pH constant 1,4, le profil de dissolution comporte une phase de latence de durée ajustable inférieure ou égale à 8 heures, de préférence inférieure ou égale à 5 heures et, plus préférentiellement encore, comprise entre 1 et 5 heures ;
   - le passage de pH 1,4 à pH 7,1 conduit à une phase de libération prolongée de durée ajustable, débutant sans temps de latence et telle que t½ est compris entre 0,25 et 20 heures, de préférence entre 0,25 et 12 heures, de préférence encore entre 0,25 et 8 heures et, plus préférentiellement encore entre 0,25 et 4 heures, où t½ est la durée nécessaire à la libération de 50% d'au moins l'un des principes actifs contenu dans les microparticles enrobées (selon la revendication 1).

Plus précisément, l'invention concerne des microparticules "réservoir" contenant au moins un principe actif (PA) et étant du type de celles :
- constituées par des particules de PA recouvertes chacune d'au moins deux pellicules d'enrobage distinctes,
- de diamètre moyen inférieur à 2000 microns et, de préférence, compris entre 50 et 800 microns et, plus préférentiellement encore, entre 100 et 600 microns ;
caractérisées en ce que chaque microparticule comprend :
- au moins une pellicule d'enrobage (A) de composition suivante :
   (A1) au moins un (co)polymère filmogène (A1) insoluble dans les liquides du tractus gastro-intestinal ;
   (A2) au moins un (co)polymère (A2) soluble dans les liquides du tractus gastro-intestinal ;
   (A3) au moins un plastifiant (A3) ;
   (A4) éventuellement au moins un agent tensioactif et/ou lubrifiant(A4) ;
- et au moins une pellicule d'enrobage (B) constituée d'un matériau composite comprenant au moins un polymère (B1) hydrophile porteur de groupements ionisés à pH neutre et au moins un composé hydrophobe (B2). selon la revendication 1.

La combinaison des deux types de pellicules d'enrobage A et B permet :
- d'assurer une libération du PA régie par deux mécanismes distincts de déclenchement, l'un basé sur une variation de pH et l'autre permettant la libération du PA, au bout d'un temps prédéterminé de résidence dans l'estomac,
- d'induire un comportement de dissolution in vitro (réalisé dans un dissolutest à palette conforme à la Pharmacopée européenne 5.2 ou appareil de type II dans l'US Pharmacopeia 28-NF23, maintenu à 37 °C et agité à 100 tours/min) tel que :
   - à pH constant 1,4, le profil de dissolution comporte une phase de latence de durée ajustable inférieure ou égale à 8 heures, de préférence inférieure ou égale à 5 heures et, plus préférentiellement encore, comprise entre 1 et 5 heures ;
   - le passage de pH 1,4 à pH 7,1 conduit à une phase de libération prolongée de durée ajustable, débutant sans temps de latence et telle que t½ compris entre 0,25 et 20 heures, de préférence entre 0,25 et 12 heures, de préférence encore entre 0,25 et 8 heures et, plus préférentiellement encore entre 0,25 et 4 heures, où t½ est la durée nécessaire à la libération de 50% d'au moins l'un des principes actifs contenu dans les microparticles enrobées.

Une caractéristique des microparticules selon l'invention est que la libération du PA est retardée et prolongée de façon contrôlée.

Avantageusement, les microparticules selon l'invention sont recouvertes de deux pellicules d'enrobage, une pellicule A et une pellicule B. La pellicule A peut être soit la couche interne soit la couche externe. Selon un mode préféré, la pellicule A est la couche interne et la pellicule B est la couche externe.

II est du mérite de la Demanderesse, d'avoir mis au point de façon tout à fait surprenante et inattendue, un tel système galénique qui associe dans des microparticules à libération modifiée de PA, deux couches de contrôle de la libération du PA, afin d'atteindre les objectifs susvisés.

Ceci était d'autant moins prévisible que l'on aurait pu craindre que se produise un mélange physique des couches A et B, du fait de la l'utilisation lors de l'enrobage de solvants communs aux deux couches. Or, un tel mélange est susceptible de perturber et de rendre incontrôlable la libération du PA hors de ces microparticules.

Il est en outre du mérite de le Demanderesse d'avoir mis au point un système qui associe deux couches, qui atteint les objectifs susvisés sans pour autant nécessiter des épaisseurs d'enrobage rédhibitoires qui entraîneraient la diminution du taux en PA des microparticules, l'augmentation des temps de préparation et les quantités de produit mises en œuvre, et donc, l'augmentation du coût.

### DESCRIPTION DETAILLEE DE L'INVENTION

Dans l'exposé de l'invention, il est fait appel au terme de "microparticules enrobées" pour désigner des microparticules de PA enrobées par au moins un revêtement permettant la libération modifiée de PA. Les microparticules de PA non enrobées (i.e. avant enrobage), peuvent être par exemple des cœurs neutres recouverts d'au moins une couche contenant du PA, ou des microparticules de PA pur ou bien encore des granules formés par une matrice d'excipients supports incluant le PA. Le terme "microparticules" recouvrira aussi bien des microparticules enrobées selon l'invention que des microparticules non enrobées.

Ces microparticules enrobées peuvent être assimilées à des véhicules permettant le transport et la libération d'au moins un PA et éventuellement d'un ou plusieurs autres principes actifs, dans l'intestin grêle voire dans le gros intestin.

Les diamètres de microparticules dont il est question dans le présent exposé sont, sauf indication contraire, des diamètres moyens en volume.

Il est particulièrement intéressant de pouvoir donner ou non aux microparticules à libération modifiée, notamment dans le cas de PA dont l'absorption intervient principalement dans les parties hautes du tractus gastro-intestinal, une période de latence de durée ajustable. Au cours de cette période de latence, la libération du PA est nulle ou quasi-nulle, grâce à la barrière étanche que forme la pellicule externe d'enrobage. Cela peut permettre de faire coïncider la libération in vivo avec le passage dans la fenêtre d'absorption propre à un PA donné.

Un autre intérêt unique d'un tel système est de permettre d'obtenir, par mélange avec une forme galénique ou des microparticules à libération immédiate, ou encore par mélange avec une autre forme galénique ou des microparticules à libération modifiée de PA, des profils de libération présentant plusieurs vagues de libération de PA (un seul ou plusieurs PA identiques ou différents) ou assurant par un réglage adéquat des différentes fractions, un niveau de concentration plasmatique du PA constant.

On ajuste le pH de déclenchement, et donc l'instant de libération du PA dans l'intestin, par une formulation appropriée de polymère(s) hydrophile(s) porteur(s) de groupements ionisés à pH neutre B1 et de composé(s) hydrophobe(s) B2 et en fonction du ratio pondéral (B2)/(B1).

Commercialement, il existe des (co)polymères (d'acide (méth)acrylique ou des phtalates de cellulose par exemple) qui provoquent le déclenchement de la libération à un pH qui peut varier de 5 à 7. Lorsque le pH de déclenchement vaut 5, la libération se produit dès la sortie de l'estomac, dans le début de l'intestin (au niveau du duodénum). Lorsque le pH de déclenchement augmente, la libération se produit de plus en plus tardivement après le passage dans l'intestin.

L'un des avantages déterminants du système galénique multimicroparticulaire, à libération retardée et contrôlée de PA, selon l'invention, est de faire intervenir in vivo deux facteurs déclencheurs de la libération du PA dans le tractus gastro-intestinal, à savoir :
o la durée de séjour dans l'estomac : libération "temps dépendante",
o la variation de pH : libération "pH dépendante".

Ces deux facteurs déclencheurs de la libération de PA agissent en parallèle, de sorte qu'ils confèrent au système galénique une grande sécurité d'emploi. La libération du PA est ainsi garantie après un temps de latence préréglé, même si la variation de pH n'est pas intervenue comme déclencheur. Les problèmes de variabilité interindividuelle (notamment, de vidange gastrique) sont ainsi surmontés. L'efficacité thérapeutique du médicament comprenant un tel système galénique est assurée, en respectant des temps prédéfinis et adaptés à la performance thérapeutique visée.

Par ailleurs, la vitesse de libération est réglée par exemple de la façon suivante :
o par le contrôle de l'épaisseur de l'enrobage A ;
o par les ratio pondéraux entre les composants A1, A2, A3 et éventuellement A4, de la couche d'enrobage A.

Suivant une caractéristique préférée des microparticules enrobées conformes à l'invention, la pellicule d'enrobage A a un taux de pelliculage (Tp_{A}) -exprimé en % en poids sec par rapport à la masse totale des microparticules enrobées- tel que TpA ≥ 2%, de préférence TpA ≥ 3% et plus préférentiellement encore TpA ≥ 4%.

Cette caractéristique correspond à une épaisseur plancher pour la couche A, en deçà de laquelle sa résistance mécanique et sa fonction de modification de la libération ne sont plus garanties.

Suivant une caractéristique préférée des microparticules enrobées conformes à l'invention, la pellicule d'enrobage A a un taux de pelliculage (Tp_{A}) -exprimé en % en poids sec par rapport à la masse totale des microparticules enrobées- inférieur ou égal à 50%.

Suivant un mode préféré de réalisation de l'invention, et s'agissant de la pellicule d'enrobage A :
* (A1) est choisi dans le groupe comprenant :
   - les dérivés non hydrosolubles de la cellulose, de préférence l'éthylcellulose et/ou l'acétate de cellulose,
   - les dérivés acryliques, par exemple les copolymères d'acide (méth)acrylique et d'ester alkyle (e.g méthyle), les copolymères d'ester d'acide acrylique et méthacrylique porteur d'au moins un groupement ammonium quaternaire (préférablement au moins un copolymère de (méth)acrylate d'alkyle et de chlorure de triméthylammonioéthyl-méthacrylate) et plus précisément les produits commercialisés sous les marques EUDRAGIT ^{®} RS et/ou RL
   - les polyvinylacétates,
   - et leurs mélanges ;
* (A2) est choisi dans le groupe comprenant :
   - les (co)polymères azotés, de préférence dans le groupe comprenant les polyacrylamides, les poly-N-vinylamides, les polyvinylpyrro-lidones (PVP) et les poly-N-vinyl-lactames ;
   - les dérivés hydrosolubles de la cellulose,
   - les alcools polyvinyliques (APV),
   - les polyoxyéthylènes (POE)
   - les polyéthylèneglycols (PEG),
   - et leurs mélanges ;
      la polyvinyl-pyrrolidone étant particulièrement préférée ;
* (A3) est choisi dans le groupe comprenant :
   - les esters de l'alcool cétylique
   - le glycérol et ses esters, de préférence dans le sous-groupe suivant : glycérides acétylés, glycérolmonostéarate, glycéryltriacétate, glycéroltributyrate,
   - les phtalates, de préférence dans le sous-groupe suivant : dibutylphthalate, diéthylphthalate, diméthylphthalate, dioctyl-phthalate,
   - les citrates, de préférence dans le sous-groupe suivant : acétyltributylcitrate, acétyltriéthylcitrate, tributylcitrate, triéthyl-citrate,
   - les sébaçates, de préférence dans le sous-groupe suivant : diéthylsébaçate, dibutylsébaçate,
   - les adipates,
   - les azélates,
   - les benzoates,
   - les huiles végétales,
   - les fumarates de préférence le diéthylfumarate,
   - les malates, de préférence le diéthylmalate,
   - les oxalates, de préférence le diéthyloxalate,
   - les succinates ; de préférence le dibutylsuccinate,
   - les butyrates,
   - les esters de l'alcool cétylique,
   - l'acide salicylique,
   - la triacétine,
   - les malonates, de préférence le diéthylmalonate,
   - l'huile de ricin (celle-ci étant particulièrement préférée),
   - et leurs mélanges ;
* (A4) est choisi dans le groupe comprenant :
   - les tensioactifs anioniques, de préférence dans le sous-groupe des sels alcalins ou alcalinoterreux des acides gras, l'acide stéarique et/ou oléique étant préférés,
   - et/ou les tensioactifs non ioniques, de préférence dans le sous-groupe suivant :
      ∘ les huiles polyoxyéthylénées de préférence l'huile de ricin hydrogénée polyoxyéthylénée,
      ∘ les copolymères polyoxyéthylène-polyoxypropylène,
      ∘ les esters de sorbitan polyoxyéthylénés,
      ∘ les dérivés de l'huile de ricin polyoxyéthylénés,
      ∘ les stéarates, de préférence de calcium, de magnésium, d'aluminium ou de zinc,
      ∘ les stéarylfumarates, de préférence de sodium,
      ∘ les béhénates de glycérol,
      ∘ et leurs mélanges.

Suivant ce mode préféré de réalisation de l'invention, et s'agissant de la pellicule d'enrobage B :
* B a un taux de pelliculage (Tp_{B}) -exprimé en % en poids sec par rapport à la masse totale des microparticules enrobées- inférieur ou égal à 50%. ;
* le ratio pondéral (B2)/(B1), est compris entre 0,2 et 1,5 , de préférence entre 0,45 et 1,0,
* le composé (B2) hydrophobe est sélectionné parmi les produits cristallisés à l'état solide et ayant une température de fusion Tf(B2) ≥ 40°C, de préférence Tf(B2) ≥ 50°C, et plus préférentiellement encore 50°C ≤ Tf(B2) ≤ 90°C.

Avantageusement, le polymère hydrophile porteur de groupements ionisés à pH neutre (B1) est choisi dans le groupe comprenant :
∘ B1.a les copolymères d'acide (méth)acrylique et d'ester alkyle (e.g méthyle) d'acide (méth)acrylique (par exemple EUDRAGIT ^{®} S ou L) ;
∘ B1.b les dérivés de la cellulose, de préférence : les acétates de cellulose, les phtalates de cellulose, les succinates de celluloses, et, plus préférentiellement encore les phtalates d'hydroxypropylméthylcellulose, les acétates d'hydroxypropylméthyl-cellulose, les succinates d'hydroxypropylméthylcellulose ;
o et leurs mélanges.

Les polymères B1 préférés sont des copolymères d'acide(méth)-acryliques et d'esters d'alkyle (e.g. alkyle en C1-C6) d'acide(méth)acrylique. Ces copolymères sont, par exemple, du type de ceux commercialisés par la société Röhm Pharma Polymers sous les marques déposées EUDRAGIT^{®}, des séries L et S (comme par exemple les EUDRAGIT^{®} L100, S100, L30 D-55 et L100-55). Ces copolymères sont des copolymères anioniques et solubles en milieu aqueux à des pH supérieurs à ceux rencontrés dans l'estomac.

Avantageusement, le composé B2 est choisi parmi le groupe de produits suivants :
o B2.a cires végétales prises à elles seules ou en mélanges entre-elles ;
o B2.b huiles végétales hydrogénées prises à elles seules ou en mélange entre-elles ;
o B2.c di et/ou tri esters du glycérol et d'au moins un acide gras ;
o B2.d et leurs mélanges.

De préférence, le composé B2 est choisi parmi le groupe de produits suivants : huile hydrogénée de graines de coton, huile hydrogénée de graines de soja, huile de palme hydrogénée, béhénate de glycérol, huile de ricin hydrogénée, tristéarine, tripalmitine, trimyristine, cire jaune, matière grasse dure ou matière grasse utile comme bases de suppositoires, matières grasses laitières anhydres, lanoline, palmitostéarate de glycérol, stéarate de glycérol, macrogolglycérides lauryliques, alcool cétylique, diisostéarate de polyglycérol, diéthylène glycol monostéarate, éthylène glycol monostéarate, Oméga 3 et tout mélange d'entre eux.

Mieux encore, le composé B2 est choisi parmi le sous-groupe de produits suivants : huile hydrogénée de graines de coton, huile hydrogénée de graines de soja, huile de palme hydrogénée, béhénate de glycérol, huile de ricin hydrogénée, tristéarine, tripalmitine, trimyristine et tout mélange d'entre eux.

En pratique et sans que cela ne soit limitatif, on préfère que le composé B2 soit choisi :
o dans le groupe de produits commercialisés sous les marques suivantes : Dynasan^{®}, Cutina^{®}, Hydrobase^{®}, Dub^{®}, Castorwax^{®}, Croduret^{®}, Compritol^{®}, Sterotex^{®}, Lubritab^{®}, Apifil^{®}, Akofine^{®}, Softtisan^{®}, Hydrocote^{®}, Livopol^{®}, Super Hartolan^{®}, MGLA^{®}, Corona^{®}, Protalan^{®}, Akosoft^{®}, Akosol^{®}, Cremao^{®}, Massupol^{®}, Novata^{®}, Suppocire^{®}, Wecobee^{®}, Witepsol^{®}, Lanolin^{®}, Incromega^{®}, Estaram^{®}, Suppoweiss^{®}, Gelucire^{®}, Precirol^{®}, Emulcire^{®}, Plurol diisostéarique^{®}, Geleol^{®}, Hydrine^{®}, Monthyle^{®} et leurs mélanges ;
o ainsi que dans le groupe d'additifs dont les codes sont les suivants : E 901, E 907, E 903 et leurs mélanges ;
o et, de préférence dans le groupe de produits commercialisés sous les marques suivantes: Dynasan^{®} P60, Dynasan^{®} 114, Dynasan^{®} 116, Dynasan^{®} 118, Cutina^{®} HR, Hydrobase^{®} 66-68, Dub^{®} HPH, Compritol^{®} 888, Sterotex^{®} NF, Sterotex^{®} K, Lubritab^{®} et leurs mélanges.

En outre, pour les PA considérés dans la présente invention dont la fenêtre d'absorption est limitée aux parties hautes du tractus gastro-intestinal, il est particulièrement avantageux que la forme à libération retardée puis prolongée soit une pluralité de microparticules enrobées. En effet, pour une telle forme, la dose de PA à administrer se répartit entre un grand nombre de microparticules enrobées (typiquement 10 000 pour une dose de 500 mg) et présente de ce fait les avantages intrinsèques suivants :
o Le temps de séjour des microparticules enrobées dans les parties hautes du tractus gastro-intestinal peut être prolongé, ce qui assure un accroissement de la durée de passage du PA devant les fenêtres d'absorption et maximise ainsi la biodisponibilité du PA.
o La mise en oeuvre d'un mélange de microparticules enrobées de profils de libération retardée et contrôlée différents, permet de réaliser des profils de libération présentant plusieurs vagues de libération ou assurant par un réglage adéquat des différentes fractions, un niveau de concentration plasmatique du PA constant.
∘ La variabilité de la vidange gastrique est moindre, car la vidange qui s'effectue ici sur un grand nombre de particules est statistiquement plus reproductible.
o On évite la mise en contact des tissus avec une dose élevée de PA (le problème du "dose dumping "). Chaque microparticule ne contient en effet qu'une dose très réduite de PA. On s'affranchit ainsi du risque de détérioration des tissus par surconcentration locale d'un PA agressif.
∘ Il est possible de présenter ces microparticules sous forme de sachet, gélule ou comprimé. Dans les cas où la dose de PA est élevée (500 mg ou plus) les formes monolithiques sont de trop grandes dimensions pour être facilement avalées. Il est alors particulièrement intéressant de disposer d'une forme microparticulaire qui assure la libération retardée et contrôlée du PA que l'homme de l'art peut mettre en forme de comprimés délitables ou de sachets.

Le système galénique multimicroparticulaire selon l'invention permet d'assurer de manière sûre une libération retardée et prolongée du PA dans le tractus gastro-intestinal, grâce à deux déclencheurs et de se soustraire ainsi à la variabilité inter et intra individuelle des conditions de la vidange gastrique, tout en étant viable économiquement et facile à ingérer (observance optimisée).

Au delà des paramètres qualitatifs de définition des microparticules enrobées selon l'invention, il peut être précisé que, conformément à une modalité quantitative de réalisation avantageuse, la pellicule d'enrobage interne de ces microparticules a la composition quantitative suivante en % en poids :
(A1) compris entre 10 et 90, de préférence entre 15 et 80,
(A2) compris entre 5 et 50, de préférence entre 10 et 40,
(A3) compris entre 1 et 30, de préférence entre 2 et 20,
(A4) compris entre 0 et 20, de préférence entre 0 et 15.

La pellicule B d'enrobage représente au plus 50 %, de préférence au plus 40 % en poids des microparticules (ou, en d'autres termes, la pellicule B a un taux de pelliculage Tp_{B} inférieur ou égal à 50 %, de préférence inférieur ou égal à 40 % en poids sec, par rapport à la masse totale de microparticules enrobées).

Selon une caractéristique préférée de l'invention, les deux pellicules d'enrobage A et B représentent ensemble au plus 50% en poids sec par rapport à la masse totale des microparticules enrobées.

Ces taux limités d'enrobage permettent de réaliser des unités galéniques contenant chacune une haute dose de principe actif, sans dépasser une taille rédhibitoire au regard de la déglutition. L'observance et donc le succès du traitement ne peuvent que s'en trouver améliorés.

Les microparticules enrobées selon l'invention comportent au moins deux pellicules d'enrobage : une pellicule interne directement au contact de la particule de principe actif, éventuellement une ou plusieurs pellicules intermédiaires, et une pellicule externe, au contact de la pellicule interne ou, le cas échéant, d'une pellicule intermédiaire.

Selon un mode de mise en œuvre particulier, la pellicule d'enrobage A est une pellicule externe et la pellicule d'enrobage B est une pellicule interne. Selon un autre mode de mise en œuvre particulier, la pellicule d'enrobage A est une pellicule interne, au contact de la particule de principe actif, et la pellicule d'enrobage B est une pellicule externe.

Le choix de la structure adéquate dépend notamment du type de principe actif, de la période de latence voulue ou de la vitesse de libération. Par exemple, pour un principe actif acide nécessitant un temps de libération long, on préfère une pellicule d'enrobage B comme pellicule interne et une pellicule d'enrobage A comme pellicule externe.

Avantageusement, les microparticules enrobées selon l'invention ne comprennent que deux pellicules d'enrobage : une pellicule d'enrobage A et une pellicule d'enrobage B. Cela permet d'atteindre les objectifs de l'invention, en particulier, le contrôle de la libération du PA en fonction du pH et du temps, ces deux mécanismes étant indépendants l'un de l'autre, tout en conférant une structure simple aux microparticules enrobées et en conservant des dimensions réduites.

S'agissant de la structure des microparticules enrobées selon l'invention, on détaille ci-après, à titre non limitatif, deux formes préférées de réalisation de cette structure.

Selon une première forme de réalisation, au moins une partie des microparticules enrobées à libération modifiée de principe(s) actif(s) comporte chacune :
o une microparticule de principe(s) actif(s), enrobée par
o au moins une pellicule d'enrobage A,
o et au moins une pellicule d'enrobage B.

De préférence, la microparticule de principe(s) actif(s) est un granulé comprenant le(les) principe(s) actif(s) et un ou plusieurs excipients pharmaceutiquement acceptables.

Selon une deuxième forme de réalisation, au moins une partie des microparticules enrobées à libération modifiée de principe(s) actif(s) comporte chacune :
o un cœur neutre,
∘ au moins une couche active comprenant le (ou les) principe(s) actif(s) et enrobant le cœur neutre,
o au moins une pellicule d'enrobage A,
o et au moins une pellicule d'enrobage B.

Suivant une première possibilité, le cœur neutre peut être par exemple un cœur neutre à base de sucre (saccharose, dextrose, lactose ou autre), une microsphère de cellulose ou toute autre particule pharmaceutiquement acceptable de diamètre moyen inférieur à 800 µm. Avantageusement, le cœur neutre a un diamètre moyen compris entre 1 et 800 µm et de préférence compris entre 20 et 500 µm.

La couche active peut éventuellement comporter, outre le (ou les) principe(s) actif(s), un ou plusieurs excipients pharmaceutiquement acceptables.

Avantageusement, les excipients pharmaceutiquement acceptables, classiques et connus de l'homme du métier, peuvent être notamment :
∘ des colorants ;
∘ des plastifiants comme par exemple le dibutylsébaçate ;
∘ des composés hydrophiles comme par exemple la cellulose et ses dérivés ou la polyvinylpyrrolidone et ses dérivés ;
∘ et leurs mélanges.

S'agissant de la préparation des microparticules enrobées, les techniques avantageusement mises en œuvre pour le dépôt du revêtement permettant la libération modifiée du(des) principe(s) actif(s) ou le dépôt de la couche active à base du(des) principe(s) actif(s), sont des techniques connues de l'homme de l'art, telles que par exemple la technique de spray coating en lit d'air fluidisé, la granulation humide, le compactage, l'extrusion-sphéronisation.

L'invention peut être mise en œuvre indépendamment de la solubilité du PA dans l'eau. Quatre classes de PA sont définies, notamment en fonction de leur solubilité, d'après le "Biopharmaceutics Classification System" (BCS) de la U.S. Food and Drug Administration : Amido G.L. et al. "A theoretical basis for a biopharmaceutics drug classification : the correlation of in vivo drug product dissolution and in vivo bioavailability", Pharmaceutical Research, vol. 12, pp. 413-420 (1995). Des PA appartenant à ces différentes classes peuvent être utilisés selon la présente invention.

Qualitativement parlant, le PA contenu dans les microparticules enrobées selon l'invention est absorbable essentiellement dans les parties hautes du tractus gastro-intestinal et il est avantageusement choisi parmi au moins l'une des familles de substances actives suivantes : les agents de traitement de l'abus d'alcool, les agents de traitement de la maladie d'Alzheimer, les anesthésiques, les agents de traitement de l'acromégalie, les analgésiques, les antiasthmatiques, les agents de traitement des allergies, les agents anticancéreux, les anti-inflammatoires, les anticoagulants et antithrombotiques, les anti-convulsivants, les antiépileptiques, les antidiabétiques, les antiémétiques, les antiglaucomes, les antihistaminiques, les anti-infectieux, les antibiotiques, les antifongiques, les antiviraux, les antiparkinsoniens, les anticholinergiques, les antitussifs, les inhibiteurs de l'anhydrase carbonique, les agents cardiovasculaires, les hypolipémiants, les anti-arythmiques, les vasodilatateurs, les antiangineux, les anti-hypertenseurs, les vasoprotecteurs, les inhibiteurs de cholinestérase, les agents de traitement des désordres du système nerveux central, les stimulants du système nerveux central, les contraceptifs, les promoteurs de fécondité, les inducteurs et inhibiteurs du travail utérin, les agents de traitement de la mucoviscidose, les agonistes des récepteurs de la dopamine, les agents de traitement de l'endométriose, les agents de traitement des dysfonctionnements érectiles, les agents de traitement de la fertilité, les agents de traitements des troubles gastro-intestinaux, les immunomodulateurs et les immunosuppresseurs, les agents de traitement des troubles de la mémoire, les antimigraineux, les relaxants des muscles, les analogues de nucléosides, les agents de traitement de l'ostéoporose, les parasympathomimétiques, les prostaglandines, les agents psychothérapeutiques, les sédatifs, les hypnotiques et tranquillisants, les neuroleptiques, les anxiolytiques, les psychostimulants, les antidépresseurs, les agents de traitements dermatologiques, les stéroïdes et les hormones.

Des agents de traitement de l'acromégalie sont par exemple : octreotide, laureotide et pegvisomant, et leurs sels, leurs esters, leurs hydrates, leurs polymorphes et leurs isomères.

Des agents de traitement de l'abus d'alcool sont par exemple : chlorazépate, chlordiazépoxyde, diazépam, disulfiram, hydroxyzine, naltrexone, et leurs sels, leurs esters, leurs hydrates, leurs polymorphes et leurs isomères.

Des anesthésiques sont par exemple : adrénaline, bupivacaïne, chloroprocaïne, desflurane, étidocaïne, levobupivacaïne, lidocaïne, midazolam, propofol, ropivacaïne, et leurs sels, leurs esters, leurs hydrates, leurs polymorphes et leurs isomères.

Des analgésiques sont par exemple : acétaminophène, aspirine, bupivacaïne, buprénorphine, butorphanol, célécoxib, clofénadol, choline, clonidine, codéine, diflunisal, dihydrocodéine, dihydroergotamine, dihydromorphine, éthylmorphine, étodolac, élétriptan, eptazocine, ergotamine, fentanyle, fénoprofène, acide hyaluronique, hydrocodone, hydromorphone, hylane, ibuprofène, indométhacine, ketorolac, kétotifène, levométhadone, levallorphane, lévorphanol, lidocaïne, acide méfénamique, méloxicam, mépéridine, méthadone, morphine, nabumétone, nalbuphine, néfopam, nalorphine, naloxone, naltrexone, naproxène, naratriptan, néfazodone, morméthadone, oxapozine, oxycodone, oxymorphone, pentazocine, péthidine, phenpyramide, piritramide, piroxicam, propoxyphène, réfécoxib, rizatriptan, kétoprofène, sulindac, sumatriptan, tébacone, tilidine, tolmétine, tramadol, zolmitriptan, et leurs sels, leurs esters, leurs hydrates, leurs polymorphes et leurs isomères.

Des antiasthmatiques sont par exemple : ablukast, azélastine, bunaprolast, cinalukast, cromitrile, cromolyne, énofélast, isambxole, kétotifène, levcromékaline, lodoxamide, montélukast, ontazolast, oxarbazole, oxatomide, piriprost potassium, pirolate, pobilukast, édamine, pranlukast, quazolast, répirinast, ritolukast, sulukast, tétrazolastméglumine, tiaramide, tibénélast, tomélukast, tranilast, verlukast, vérofylline, zarirlukast, et leurs sels, leurs esters, leurs hydrates, leurs polymorphes et leurs isomères.

Des agents anticancéreux sont par exemple : adriamycine, aldesleukine, allopurinol, altrétamine, amifostine, anastrozole, asparaginase, bétaméthasone, bexarotène, bicalutamide, bléomycine, busulfan, capécitabine, carboplatine, carmustine, chlorambucil, cisplatine, cladribine, œstrogène conjugué, cortisone, cyclophosphamide, cytarabine, dacarbazine, daunorubicine, dactinomycine, dénileukine, dexaméthasone, discodermolide, docétaxel, doxorubicine, éloposidem, épirubicine, époétine, épothilones, estramustine, œstrogène estérifié, éthinyl-œstradiol, étoposide, exemestane, flavopirdol, fluconazole, fludarabine, fluorouracile, flutamide, floxuridine, gemcitabine, gemtuzumab, goséréline, hexaméthylmélamine, hydrocortisone, hydroxyurée, idarubicine, ifosfamide, interféron, irinotécan, lémiposide, létrozole, leuprolide, lévamisole, lévothyroxine, lomustine, méchloréthamine, melphalan, mercaptopurine, mégestrol, méthotrexate, méthylprednisolone, méthyltestosterone, mithramycine, mitomycine, mitotane, mitoxantrone, mitozolomide, mutamycine, nilutamide, paclitaxel, pamidronate, pegaspargase, pentostatine, plicamycine, porfimer, prednisolone, procarbazine, rituximab, sargramostim, sémustine, streptozocine, tamoxifen, témozolamide, téniposide, testolactone, thioguanine, thiotépa, tomudex, topotécan, torémifène, trastumuzab, trétinoïne, sémustine, streptozolocine, valrubicine, verteprofine, vinblastine, vincristine, vindesine, vinorelbine, et leurs sels, leurs esters, leurs hydrates, leurs polymorphes et leurs isomères.

Des anticoagulants et des antithrombotiques sont par exemple : warfarine, daltéparine, héparine, tinzaparine, énoxaparine, danaparoïde, abciximab, alprostadil, altiplase, anagralide, anistreplase, argatroban, ataprost, bétaprost, camonagrel, cilostazol, clinprost, clopidogrel, cloricromène, dermatan, désirudine, domitroban, drotavérine, époprosténol, éptifibatide, fradafiban, gabexate, iloprost, isbogrel, lamifiban, lamotéplase, léfradafiban, lépirudin, lévosimendan, lexipafant, mélagatran, nafagrel, nafamostsat, nizofenone, orbifiban, ozagrel, pamicogrel, parnaparin, quinobendan, réteplase, sarpogralate, satigrel, silteplase, simendan, ticlopidine, vapiprost, tirofiban, xemilofiban, Y20811, et leurs sels, leurs esters, leurs hydrates, leurs polymorphes et leurs isomères.

Des anticonvulsivants sont par exemple : carbamazépine, clonazepam, clorazépine, diazépam, divalproex, éthosuximide, éthotion, felbamate, fosphénytoïne, gabapentine, lamotrigine, lévétiracétam, lorazépam, méphénytoïne, méphobarbital, métharbital, méthsuximide, oxcarbazépine, phénobarbital, phénytoïne, primidone, tiagabine, topiramate, acide valproïque, vigabatrine, zonisamide, et leurs sels, leurs esters, leurs hydrates, leurs polymorphes et leurs isomères.

Des antidiabétiques sont par exemple : acarbose, acétohexamide, carbutamide, chlorpropamide, épalrestat, glibornuride, gliclazide, glimépiride, glipizide, gliquidone, glisoxepide, glyburide, glyhexamide, metformine, miglitol, natéglinide, orlistat, phénbutamide, pioglitazone, répaglinide, rosiglitazone, tolazamide, tolbutamide, tolcyclamide, tolrestat, troglitazone, voglibose, et leurs sels, leurs esters, leurs hydrates, leurs polymorphes et leurs isomères.

Des antiémétiques sont par exemple : alprazolam, benzquinamide, benztropine, bétahistine, chlorpromazine, dexaméthasone, difénidol, dimenhydrinate, diphénhydramine, dolasétron, dompéridone, dronabinol, dropéridol, granisétron, halopéridol, lorazépam, meclizine, méthylprednisolone, métoclopramide, ondansétron, perphénazine, prochlorpérazine, promethazine, scopolamine, tributine, triéthylpérazine, triflupromazine, triméthobenzamide, tropisétron, et leurs sels, leurs esters, leurs hydrates, leurs polymorphes et leurs isomères.

Des agents antiglaucome sont par exemple : alprénoxime, dapiprazole, dipivéfrine, latanoprost, naboctate, pimabine, et leurs sels, leurs esters, leurs hydrates, leurs polymorphes et leurs isomères.

Des antihistaminiques sont par exemple : acépromazine, acrivastine, activastine, albutérol, alimémazine, antazoline, azélastine, bitoltérol, amlexanox, benzydamine, bromphéniramine, cétirizine, chlorphéniramine, cimétidine, cinnarizine, clémastine, clofédanol, cycloheptazine, cyproheptadine, diclofénac, difencloxazine, diphénhydramine, dotarizine, éphédrine, épinastine, épinéphrine, éthylnorépinéphrine, étybenzatropine, fenpentadiol, fenpotérol, fexofénadine, flurbiprofène, hydroxyzine, isoétharine, isoprotérénol, bromure d'ipratropium, kétorolac, lévocetirizine, lévomépromazine, loratidine, méquitazine, métaprotérénol, niaprazine, oxatomide, oxomémazine, phényléphrine, phénylpropanolamine, pirbutérol, prométhazine, pseudoéphédrine, pyrilamine, salmétérol, terbutaline, terfénadine, tranilast, dérivés de la xanthine, xylométazoline, et leurs sels, leurs esters, leurs hydrates, leurs polymorphes et leurs isomères.

Des agents anti-infectieux, notamment les antibiotiques, les antifongiques, les antiviraux, sont par exemple : abacavir, acyclovir, albendazole, amantadine, amphotéricine, amikacine, acide aminosalicylique, amoxycilline, ampicilline, amprénavir, atovaquine, azithromycine, aztréonam, carbenicilline, céfaclor, céfadroxil, céfamandole, céfazolin, cefdinir, céfépime, céfexime, céfopérazone, céfotaxime, céfotitam, céfopérazone, céfoxitine, cefpodoxine, cefprozil, ceftazidime, ceftibutène, ceftizoxime, ceftriaxone, céfuroxime, céphalexine, chloroquine, cidofovir, cilastatine, ciprofloxacine, clarithromycine, acide clavulanique, clindamycine, colistiméthate, dalfopristine, dapsone, daunorubicine, delavirdine, déméclocycline, didanosine, doxycycline, doxorubicine, éfavirenz, énoxacine, érythromycine, éthambutol, éthionamide, famcyclovir, fluconazole, flucytocine, foscarnet, fosfomycine, ganciclovir, gatifloxacine, griseofulvine, hydroxychloroquine, imipenem, indinavir, interféron, isoniazide, itraconazole, ivermectil, kétoconazole, lamivudine, lévofloxacine, linizolide, loméfloxacine, loracarbef, mébendazole, méfloquine, méropénem, méthanamine, métronidazole, minocycline, moxefloxacine, acide naldixique, nelfinavir, néomycine, névirapine, nitorfurantoïne, norfloxacine, ofloxacine, oseltamivir, oxytetracycline, palivizumab, pénicilline, perfloxacine, pipéracilline, praziquantel, pyrazinamide, pyriméthamine, quinidine, quinupristine, rétonavir, ribavirine, rifabutine, rifampicine, rimantadine, saquinavir, sparfloxacine, stavudine, streptomycine, sulfamethoxazole, tétramycine, terbinafine, tétracycline, ticarcillin, thiabendazole, tobramycine, triméthoprim, trimétraxate, troléandomycine, trovafloxacine, valacyclovir, vancomycine, zalcitabine, zanamivir, zidovudine, et leurs sels, leurs esters, leurs hydrates, leurs polymorphes et leurs isomères.

Des antiparkinsoniens sont par exemple : amantadine, adrogolide, altinicline, benzatropine, bipéridène, brasofensine, bromocriptine, budipine, cabergoline, CHF-1301, dihydrexidine, entacapone, etilevodopa, idazoxane, iométopane, lazabémide, melevodopa, carbidopa, levodopa, mofégiline, moxiraprine, pergolide, pramipexole, quinélorane, rasagiline, ropinirole, séligiline, talipexole, tolcapone, trihexyphenidyle, et leurs sels, leurs esters, leurs hydrates, leurs polymorphes et leurs isomères.

Des agents antirhumatismants sont par exemple : azathiprine, bétaméthasone, célécoxib, cyclosporine, diclofénac, hydroxychloroquine, indométhacine, infliximab, acide mercaptobutanedioïque, méthylprednisolone, naproxène, pénicillamine, piroxicam, prednisolone, sulfasalazine, et leurs sels, leurs esters, leurs hydrates, leurs polymorphes et leurs isomères.

Des anti-agrégeants plaquettaires sont par exemple : abciximab, anagrélide, aspirine, cilostazol, clopidogrel, dipyridamole, époprosténol, éptifibatide, ticlopidine, tinofiban, et leurs sels, leurs esters, leurs hydrates, leurs polymorphes et leurs isomères.

Des antispasmodiques et d'anticholinergiques sont par exemple : aspirine, atropine, diclofénac, hyoscyamine, mésoprostol, méthocarbamol, phénobarbital, scopolamine, et leurs sels, leurs esters, leurs hydrates, leurs polymorphes et leurs isomères.

Des antitussifs sont par exemple : acétaminophène, acrivastine, albutérol, benzonatate, béractant, bromphéniramine, caféine, calfactant, carbétapentane, chlorphéniramine, codéine, colfuscérine, dextromethorpham, dornase alpha, doxylamine, épinephrine, fexofénadine, guaïfénésine, ipratropium, lévalbutérol, métaprotérénol, montélukast, pentoxyphilline, phényléphrine, phénylpropanolamine, pirbutérol, poractant alpha, pseudoéphédrine, pyrilamine, salbuterol, salmeterol, terbutaline, theophylline, zafirlukast, zileuton et leur sels, leurs esters, leurs hydrates, leurs polymorphes et leurs isomères.

Des inhibiteurs de l'anhydrase carbonique sont par exemple : acétazolamide, dichlorphénamide, dorzolamide, méthazolamide, sézolamide, et leurs sels, leurs esters, leurs hydrates, leurs polymorphes et leurs isomères.

Des agents cardiovasculaires, notamment les hypolipémiants, les anti-arythmiques, les vasodilatateurs, les antiangineux, les anti-hypertenseurs, les vasoprotecteurs, sont par exemple : abciximab, acébutolol, activase, adénosine, adrénaline, amidarone, amiloride, amlodipine, nitrate d'amyle, aténolol, atorvastatine, benzépril, bépiridil, bétaxalol, bisoprolol, candésartan, captopril, carténolol, carvédilol, cérivastatine, chlorthalidone, chlorthiazole, clofibrate, clonidine, colestipol, colosévélam, digoxine, diltiazem, disopyramide, dobutamine, dofétilide, doxazosine, énalapril, époprostenol, éprosartan, esmolol, éthacrynate, érythrityl, félodipine, fénoidapam, fosinopril, flécaïnide, flurosémide, fluvastatine, gemfibrozil, hydrochlorthiazide, hydroflumethazine, ibutilide, indapamide, isosorbide, irbésartan, labétolol, lacidipine, lisinopril, losartan, lovastatine, mécamylamine, métoprolol, métarminol, métazolone, méthylchlothiazide, méthyldopa, métyrosine, mexilétine, midrodine, milrinone, moexipril, nadolol, niacine, nicardipine, nicorandil, nifédipine, nimodipine, nisoldipine, nitroglycérine, phénoxybenzamine, périndopril, polythiazide, pravastatine, prazosine, procaïnamide, propafénone, propranolol, quanfacine, quinapril, quinidine, ranipril, rétéplase, simvastatine, sotalol, spironolactone, streptokinase, telmisartan, térazosine, timolol, tocaïnamide, torsémide, trandolapril, triamtérène, trapidil, valsartan, et leurs sels, leurs esters, leurs hydrates, leurs polymorphes et leurs isomères.

Des vasodilatateurs sont par exemple : adénosine, alvérine, caféine, dihydroergocornine, énalapril, énoximone, iloprost, kalléone, lidoflazine, nicardipine, nimodipine, acide nicotinique, papaverine, pilocarpine, salbutamol, théophylline, trandolapril, uradipil, vincamine, et leurs sels, leurs esters, leurs hydrates, leurs polymorphes et leurs isomères.

Des inhibiteurs de cholinestérase sont par exemple : donépezil, édrophonium, néostigmine, pyridostigmine, rivastigmine, tacrine, et leurs sels, leurs esters, leurs hydrates, leurs polymorphes et leurs isomères.

Des stimulants du système nerveux central sont par exemple : caféine, doxapram, dexoamphétamine, donépézil, édorphonium, méthamphétamine, méthylphénidate, modafinil, néostigmine, pémoline, phentermine, pyridostigmine, rivastigmine, tacrine et leur sels, leurs esters, leurs hydrates, leurs polymorphes et leurs isomères.

Des contraceptifs sont par exemple : désogestral, éthinyl-œstradiol, éthynodiol, lévonorgestrel, médroxyprogestérone, mestranol, norgestimate, noréthindrone, norgestrel, et leurs sels, leurs esters, leurs hydrates, leurs polymorphes et leurs isomères.

Des agents de traitement de la mucoviscidose sont par exemple : domase alpha, pancrélipase, tobramycine, et leurs sels, leurs esters, leurs hydrates, leurs polymorphes et leurs isomères.

Des agonistes des récepteurs de la dopamine sont par exemple : amantadine, cabergoline, fenoldopam, pergolide, pramipezal, ropinirole et leur sels, leurs esters, leurs hydrates, leurs polymorphes et leurs isomères.

Des agents de traitement de l'endométriose sont par exemple : danazol, goséréline, leuprolide, nafaréline, norethindrone, et leurs sels, leurs esters, leurs hydrates, leurs polymorphes et leurs isomères.

Des agents de traitement des disfonctionnements érectiles sont par exemple alprostadil, sildénafil, yohimbine, et leurs sels, leurs esters, leurs hydrates, leurs polymorphes et leurs isomères.

Des agents de traitement de la fertilité sont par exemple : citrorélix, clomiphène, follitropine, ganirélix, gonadotropine, ménotropine, progésterone, urofollitropine, et leurs sels, leurs esters, leurs hydrates, leurs polymorphes et leurs isomères.

Des agents de traitement de troubles gastro-intestinaux sont par exemple : alosétron, bisacodyl, subsalicylate de bismuth, célécoxib, cimétidine, difoxine, diphéoxylate, docusate, ésoméprazole, famotidine, glycopyrrolate, infliximab, lansoprazole, lopéramide, métaclopramide, nizatidine, oméprazole, pantoprazole, rabéprazole, ranitidine, siméthicone, sucralfate, et leurs sels, leurs esters, leurs hydrates, leurs polymorphes et leurs isomères.

Des immunomodulateurs et des immunosuppresseurs sont par exemple : azathioprine, ceftizoxine, cyclosporine, daclizumab, glatiramer, immunoglobuline, interféron, leflunomide, lévamisol, mycophénolate, phthalidomide, ribavirine, sirolimus, et leurs sels, leurs esters, leurs hydrates, leurs polymorphes et leurs isomères.

Des agents de traitements de la maladie d'Alzheimer sont par exemple : CP 118954, donépezil, galanthamine, métrifonate, révastigmine, tacrine, TAK-147, et leurs sels, leurs esters, leurs hydrates, leurs polymorphes et leurs isomères.

Des antimigraineux sont par exemple : acétaminophène, dihydroergotamine, divalproex, ergotamine, propranolol, risatriptan, sumatriptan, trimetrexate, et leurs sels, leurs esters, leurs hydrates, leurs polymorphes et leurs isomères.

Des relaxants des muscles sont par exemple : chlorure d'alcuronium, azapropazone, atracurium, baclofène, carisoprodol, dérivés de quinine, chloromézanone, chlorophénésincarbamate, chlorozoxazone, cyclobenzaprine, dantrolène, bromure de décaméthonium, chlorure de dimethyltubocurarinium, doxacurium, fényramidol, triethiodure de gallamine, guaïfénésine, bromure d'hexafluorenium, bromure d'hexacarbacholine, mémantin, méphénésine, méprobamate, métamisol, métaxalone, méthocarbamol, mivacurium, orphénadrine, pancuronium, phénazone, phénprobamate, pipécuronium, rapacuronium, rocuronium, succinylcholine, chlorure de suxaméthonium, tétrazépam, tizanidine, chlorure de tubocurarine, tybamate, vecuronium, et leurs sels, leurs esters, leurs hydrates, leurs polymorphes et leurs isomères.

Des analogues de nucléoside sont par exemple : abacavir, acyclovir, didanosine, gamciclovir, gemcitabine, lamivudine, ribavirine, stavudine, zalcitabine, et leurs sels, leurs esters, leurs hydrates, leurs polymorphes et leurs isomères.

Des agents de traitement de l'ostéoporose sont par exemple : alendronate, calcitonine, œstradiol, estropipate, médroxyprogestérone, noréthindrone, norgestimate, pamidronate, raloxifène, risdronate, zolédronate, et leurs sels, leurs esters, leurs hydrates, leurs polymorphes et leurs isomères.

Des parasympathomimétiques sont par exemple : béthanechol, bipéridine, édrophonium, glycopyrolate, hyoscyamine, pilocarpine, tacrine, yohimbine, et leurs sels, leurs esters, leurs hydrates, leurs polymorphes et leurs isomères.

Des prostaglandines sont par exemple : alprostadil, époprosténol, misoprostol, et leurs sels, leurs esters, leurs hydrates, leurs polymorphes et leurs isomères.

Des agents psychothérapeutiques sont par exemple : acétophénazine, alentémol, alpertine, alprazolam, amitriptyline, apriprazole, azapérone, batélapine, béfipiride, benpéridol, benzindopyrine, bimithil, biripérone, brofoxine, brompéridol, bronipéridol, bupropione, buspirone, butaclamol, butapérazine, butapérazin, carphénazine, carvotroline, cériclamine, chlorazépine, chlordiazépoxide, chlorpromazine, chlorprothixène, cinpérène, cintriamide, citalopram, clomacrane, clonazépam, clopenthixol, clopimozide, clopipazane, cloropérone, clothiapine, clothixamide, clozapine, cyclophénazine, dapiprazole, dapoxétine, désipramine, divalproex, dipyridamole, doxépine, dropéridol, duloxétine, eltoprazine, eptipirone, étazolate, fénimide, flibansérine, flucindole, flumézapine, fluoxétine, fluphénazine, fluspipérone, fluspirilène, flutroline, fluvoxamine, gépirone, gévotroline, halopémide, halopéridol, hydroxyzine, hydroxynortriptyline, ilopéridone, imidoline, lamotrigine, loxapine, enpérone, mazapertine, méphobarbital, méprobamate, mésoridazine, mésoridazine, milnacipran, mirtazépine, métiapine, milenpérone, milipertine, molindone, nafadotride, naranol, nefazodone, neflumozide, ocapéridone, odapipam, olanzapine, oxethiazine, oxipéromide, pagoclone, palipéridone, paroxitène, penfluridol, pentiapine, perphénazine, phénelzine, pimozide, pinoxépine, pipampérone, pipéracétazine, pipotiazine, piquindone, pirlindole, pivagabine, pramipexole, prochlorpérazine, promazine, quétiapine, réboxetine, rémoxipride, rispéridone, rimcazole, robolzotan, sélégiline, sépéridol, sertraline, sertindole, seteptiline, sétopérone, spipérone, sunipitrone, tépirindole, thioridazine, thiothixène, tiapride, tiopéridone, tiospirone, topiramate, tranylcypromine, trifluopérazine, triflupéridol, triflupromazine, trimipramine, venlafaxine, ziprasidone, et leurs sels, leurs esters, leurs hydrates, leurs polymorphes et leurs isomères.

Des sédatifs, des hypnotiques et des tranquillisants sont par exemple : bromazépam, buspirone, clazolam, clobazam, chlorazépate, diazépam, démoxépam, dexmédétomidine, diphényhydramine, doxylamine, enciprazine, estrazolam, hydroxyzine, ketazolam, lorazatone, lorazépam, loxapine, médazépam, mépéridine, méthobarbital, midazolam, nabilone, nisobamate, oxazépam, pentobarbital, prométhazine, propofol, triazolam, zaléplon, zolpidem, et leurs sels, leurs esters, leurs hydrates, leurs polymorphes et leurs isomères.

Des agents de traitement dermatologiques sont par exemple : acitrétine, alclométasone, alitretinoïne, bétaméthasone, calciprotrine, chlorhexidine, clobétasol, clocortolone, clotriamozole, collagénase, cyclosporine, désonide, difluorosone, doxépine, eflomithine, finastéride, fluocinolone, flurandrénolide, fluticasone, halobétasol, hydrochloroquine, hydroquinone, hydroxyzine, kétoconazole, mafénide, malathion, ménobenzone, néostigmine, nystatine, podofilox, povidone, tazorotène, trétinoïne, et leurs sels, leurs esters, leurs hydrates, leurs polymorphes et leurs isomères.

Des stéroïdes et des hormones sont par exemple : alclométasone, bétaméthasone, calcitonine, citrorélix, clobétasol, clocortolone, les cortisones, danazol, desmopressine, désonide, désogestrel, désoximétasone, dexaméthasone, diflorasone, œstradiol, œstrogènes, estropipate, éthynilestradiol, fluocinolone, flurandrénolide, fluticasone, glucagon, gonadotropine, goséréline, halobétasol, hydrocortisone, leuprolide, lévonorgestrel, lévothyroxine, médroxyprogestérone, les ménotropines, méthylprednisolone, méthyltestosterone, mométasone, naféréline, norditropine, noréthindrone, norgestrel, octréolide, oxandrolone, oxymétholone, polytropine, prednicarbate, prednisolone, progestérone, sermoréline, somatropine, stanozolol, testostérone, urofollitropine, et leurs sels, leurs esters, leurs hydrates, leurs polymorphes et leurs isomères.

On peut également se référer à la liste de principes actifs donnés dans la demande EP-A-0 609 961 aux pages 4 à 8.

Selon un autre de ses aspects, l'invention concerne un médicament ou une formulation pharmaceutique, vétérinaire ou diététique caractérisée en ce qu'elle comprend une pluralité de microparticules enrobées telles que définies ci-dessus, par exemple, au moins 500, de préférence de 1.000 à 1.000.000, et, plus préférentiellement encore, de 5.000 à 500.000 microparticules.

Le médicament selon l'invention est donc multimicroparticulaire, c'est-à-dire qu'il comprend au moins des microparticules de principe(s) actif(s) enrobées. Ces microparticules de principe(s) actif(s) peuvent être, par exemple, du(des) principe(s) actif(s) brut(s) (pur(s)) sous forme pulvérulente, des granules matriciels de principe(s) actif(s) avec différents autres ingrédients ou bien encore des microsphères neutres recouvertes d'au moins une couche comportant du principe actif, comme cela est expliqué ci-dessus.

Les microparticules de PA enrobées à libération modifiée sont assimilables à des micro-unités contenant au moins un principe actif et formant au moins une partie des éléments constitutifs du médicament selon l'invention.

Ces microparticules sont d'autant plus intéressantes qu'elles sont en outre parfaitement tolérées par l'organisme, notamment au niveau gastrique et par ailleurs peuvent être obtenues de façon aisée et économique.

Chaque microparticule enrobée peut comprendre un ou plusieurs principes actifs.

Le médicament ou la formulation selon l'invention peut comprendre au moins un principe actif sous une forme à libération immédiate, par exemple des micro-unités de principe actif autres que des microparticules enrobées. Il pourrait s'agir, par exemple, de microparticules à libération immédiate de principe(s) actif(s). Ces dernières peuvent être par exemple des microparticules de principe(s) actif(s) non enrobées du même type que celles utiles dans la préparation des microparticules enrobées selon l'invention.

Le principe actif sous une forme à libération immédiate peut être identique ou différent du ou des principe(s) actif(s) contenu(s) dans les microparticules enrobées. Chaque microparticule à libération immédiate peut comprendre un ou plusieurs principes actifs.

Ainsi, selon une variante de l'invention, le médicament ou la formulation comprend au moins un principe actif sous une forme à libération immédiate qui est le même qu'un principe actif contenu dans au moins une partie des microparticules enrobées.

En outre, l'ensemble des micro-unités (microparticules et/ou microparticules enrobées) constituant le médicament selon l'invention peut être formé par différentes populations de micro-unités, ces populations différant entre elles au moins par la nature du(ou des) principe(s) actif(s) contenu(s) dans ces micro-unités et/ou par la quantité en principe(s) actif(s) éventuel(s) qu'elles contiennent et/ou par la composition des pellicules d'enrobage A et/ou B et/ou par le fait qu'elles sont à libération modifiée ou à libération immédiate.

L'invention vise ainsi un médicament ou une formulation pharmaceutique, vétérinaire ou diététique comprenant une pluralité de populations de microparticules, (enrobées ou non enrobées) lesdites populations se distinguant les unes des autres par leur temps de latence et/ou par leur pH de déclenchement et/ou par leur vitesse de libération et/ou par le principe actif qu'elles contiennent.

Sans vouloir être limitatif, il doit être néanmoins souligné que le médicament selon l'invention est particulièrement intéressant en ce qu'il peut se présenter :
o sous forme de dose unique orale journalière comprenant de 500 à 500.000 micro-unités dont certaines contiennent du(des) principe(s) actif(s) ;
o sous forme de dose unique orale journalière comprenant de 500 à 500.000 microparticules (enrobées et/ou non enrobées) à libération modifiée du(des) principe(s) actif(s).

Avantageusement, la formulation pharmaceutique, vétérinaire ou diététique comprenant des microparticules enrobées selon l'invention, est sous une forme galénique choisie dans le groupe comprenant : les comprimés (avantageusement orodispersibles ou gastrodispersibles), les poudres, les suspensions, les sirops, les poudres pour suspension à reconstituer ou les gélules.

Il peut être intéressant de mélanger dans une même gélule, un même comprimé ou une même poudre, au moins deux types de microparticules enrobées à cinétiques de libération différentes mais comprises dans le cadre caractéristique de l'invention.

On peut également mélanger les microparticules enrobées selon l'invention avec une certaine quantité de PA immédiatement disponible dans l'organisme. Il est également envisageable d'associer des microparticules enrobées contenant des PA différents.

L'invention concerne également l'utilisation des microparticules enrobées décrites ci-dessus pour la fabrication de nouveaux médicaments ou préparations pharmaceutiques, vétérinaires ou diététiques de divers PA, ayant des performances thérapeutiques ou diététiques optimisées et se présentant de préférence sous forme de comprimés avantageusement orodispersibles ou gastrodispersibles, de poudres ou de gélules.

La présente invention concerne, en outre, ces nouvelles préparations pharmaceutiques, vétérinaires ou diététiques en tant que telles, originales dans leur structure, leur présentation et leur composition. De telles préparations pharmaceutiques, vétérinaires ou diététiques sont administrées per os, de préférence par doses journalières uniques.

Enfin, l'invention vise également un procédé de traitement thérapeutique caractérisé en ce qu'il consiste en une ingestion selon une posologie déterminée, d'un médicament comprenant les microparticules enrobées telles que définies ci-dessus.

L'invention sera mieux expliquée par les exemples ci-après, donnés uniquement à titre d'illustration et permettant de bien comprendre l'invention et de faire ressortir ses variantes de réalisation et/ou de mise en oeuvre, ainsi que ses différents avantages.

### BREVE DESCRIPTION DES FIGURES

La figure 1 représente les profils de libération in vitro des microparticules enrobées de l'exemple 2. Les profils sont des courbes en pourcentage en poids (% dissous) de metformine HCl dissoute en fonction du temps T en heures. à pH=7,1 : 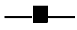 à pH=1,4 : 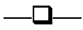 à pH évolutif : - - -O- - -

La figure 2 représente les profils de libération in vitro des microparticules enrobées de l'exemple 3. Les profils sont des courbes en pourcentage en poids (% dissous) de metformine HCl dissoute en fonction du temps T en heures. à pH=7,1 : 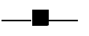 à pH=1,4 : 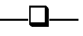 à pH évolutif : - - -O- - -

La figure 3 représente les profils de libération in vitro des microparticules enrobées de l'exemple 4. Les profils sont des courbes en pourcentage en poids (% dissous) de metformine HCl dissoute en fonction du temps T en heures. à pH=7,1 : 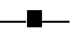 à pH=1,4 : 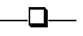 à pH évolutif : - - -O- - -

La figure 4 représente les profils de libération in vitro à pH évolutif, des exemples 2, 3 et 4. Les profils sont des courbes en pourcentage en poids (% dissous) de metformine HCl dissoute en fonction du temps T en heures. à pH évolutif : exemple 2 : 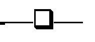 exemple 3 : 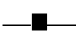 exemple 4 : - - -O- - -

La figure 5 représente les profils de libération in vitro des microparticules enrobées de l'exemple 5. Les profils sont des courbes en pourcentage en poids (% dissous) de metformine HCl dissoute en fonction du temps T en heures.
à pH=7,1 : 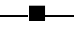 à pH=1,4 :

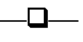

La figure 6 représente les profils de libération in vitro des microparticules enrobées de l'exemple 6. Les profils sont des courbes en pourcentage en poids (% dissous) d'acyclovir dissous en fonction du temps T en heures.
à pH=7,1 : 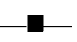 à pH=1,4 :

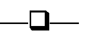

### EXEMPLES

Les exemples 1 à 5 mettent en œuvre la metformine HCl, qui est un PA fortement soluble dans l'eau. L'exemple 6 met en œuvre l'acyclovir, qui est un PA faiblement soluble dans l'eau.

L'exemple 2 est un exemple comparatif de préparation de microparticules enrobées qui ne comportent qu'une couche d'enrobage B. Dans les exemples 3, 4 et 6, on prépare des microparticules enrobées qui comportent une couche d'enrobage interne A et une couche d'enrobage externe B. Dans l'exemple 5, on prépare des microparticules enrobées qui comportent une couche d'enrobage interne B et une couche d'enrobage externe A.

Dans les exemples suivants les noms commerciaux des excipients cités trouvent leur correspondance chimique dans le tableau suivant :

| Nom commercial | Nom chimique / Monographie |
|---|---|
| Cremophor RH 40 | Macrogolglyceroli hydroxystearas |
| Klucel EF | Hydroxypropyl cellulose |
| Plasdone K29/32 | Povidone |
| Eudragit L100-55 | Poly(methacrylique acide, ethyl acrylate) 1 :1 |
| Kollicoat MAE 100P | Poly(methacrylique acide, ethyl acrylate) 1 :1 |
| Acrycoat L100D | Poly(methacrylique acide, ethyl acrylate) 1 :1 |
| Eudragit S100 | Poly(methacrylique acide, methyl methacrylate) 1 :2 |
| Ethocel 20P | Ethylcellulose |

Dans les exemples, les tests de dissolution sont réalisés dans un dissolutest à palettes conforme à la Pharmacopée européenne 5.2 ou appareil de type II dans l'US Pharmacopeia 28-NF 23, maintenu à 37 °C et agité à 100 tours/min.

### EXEMPLE 1 : Préparation de granulés de metformine, HCl

1795,5g de metformine, HCl (Chemsource) et 94,5g de povidone sont dissous dans 2610g d'eau. La solution est pulvérisée sur 210g de microsphères neutres (NP Pharm) dans un spray coater Glatt^{®} GPCG3.

### EXEMPLE 2 : Exemple selon l'art antérieur de microparticules enrobées conduisant à une libération retardée de Metformine, HCl (exemple comparatif).

On dissout à chaud 78,0g d'huile végétale hydrogénée Type 1 NF (JRS Pharma) et 117,0g d'Eudragit^{®}L100-55 (Röhm) dans 1756,0g d'éthanol. 1140g de cette solution sont pulvérisés sur 455,0g des microgranulés de metformine, HCl préparés dans l'exemple 1 dans un spray coater Glatt^{®} GPCG3. On obtient des microparticules enrobées.

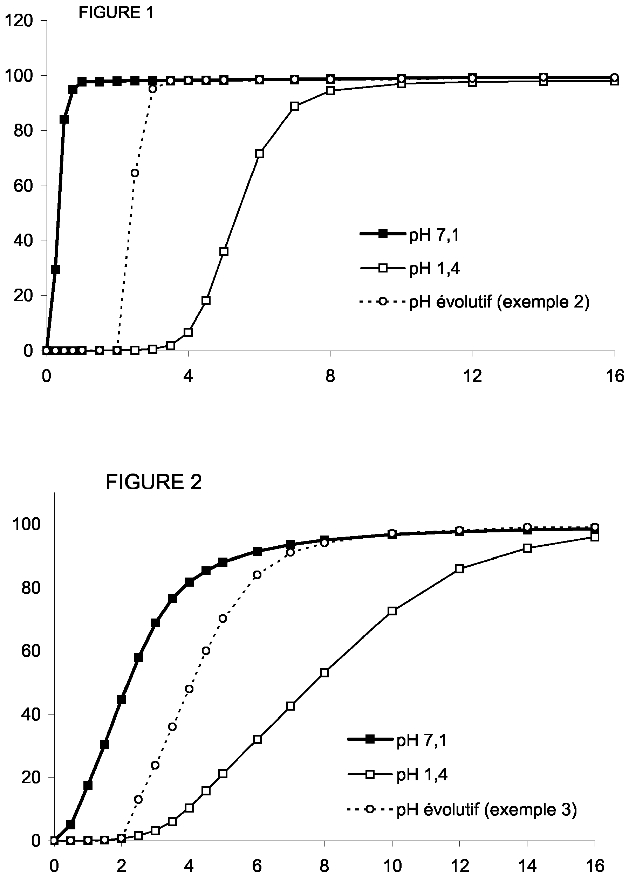

On a réalisé des tests de dissolutions sur les microparticules enrobées dans les milieux suivants : i) solution d'HCl à pH 1,4 ii) solution tamponnée KH₂PO₄/NaOH à pH 7,1 et iii) solution d'HCl à pH 1,4 2h puis en milieu tampon KH₂PO₄/NaOH à pH 7,1. Les résultats des tests de dissolution sont représentés en figure 1.

### EXEMPLE 3 : Préparation selon l'invention de microparticules enrobées conduisant à une libération retardée et prolongée de metformine, HCl.

On pellicule 517g des microgranulés de l'exemple 1, avec 632g de la solution suivante : ethylcellulose 24,4g, povidone 2,6g, stéarate de magnésium 3,3g, huile de ricin 2,6g, éthanol 627g.

On dissout à chaud 45,5g d'huile végétale hydrogénée Type 1 NF (JRS Pharma) et 68,3g d'Eudragit^{®}L100-55 (Röhm) dans 1024g d'éthanol. 650g de cette solution sont ensuite pulvérisés sur 455g des microparticules précédemment obtenues. Le pelliculage s'effectue dans un spray coater Glatt^{®}. On obtient des microparticules enrobées.

On a réalisé des tests de dissolutions sur les microparticules enrobées dans les milieux suivants : i) solution d'HCl à pH 1,4 ii) solution tamponnée KH₂PO₄/NaOH à pH 7,1 et iii) solution d'HCl à pH 1,4 2h puis en milieu tampon KH₂PO₄/NaOH à pH 7,1. Les résultats des tests de dissolution sont représentés en figure 2.

### EXEMPLE 4 : Préparation selon l'invention de microparticules enrobées conduisant à une libération retardée et prolongée de metformine, HCl.

On pellicule 500g des microgranulés de l'exemple 1 avec 995g de la solution suivante : ethylcellulose 60,8g, Povidone 3,2g, Stéarate de magnésium 8,0g, Huile de ricin 8,0g, Ethanol 925g.

On dissout à chaud 45,5g d'huile végétale hydrogénée Type 1 NF (JRS Pharma) 22,8g d'Eudragit^{®}L100-55 (Röhm) et 45,5 d'Eudragit^{®}S100 (Röhm) dans 1024g d'éthanol. 650g de cette solution sont ensuite pulvérisés sur 455g des microparticules précédemment obtenues. Le pelliculage s'effectue dans un spray coater Glatt^{®}. On obtient des microparticules enrobées.

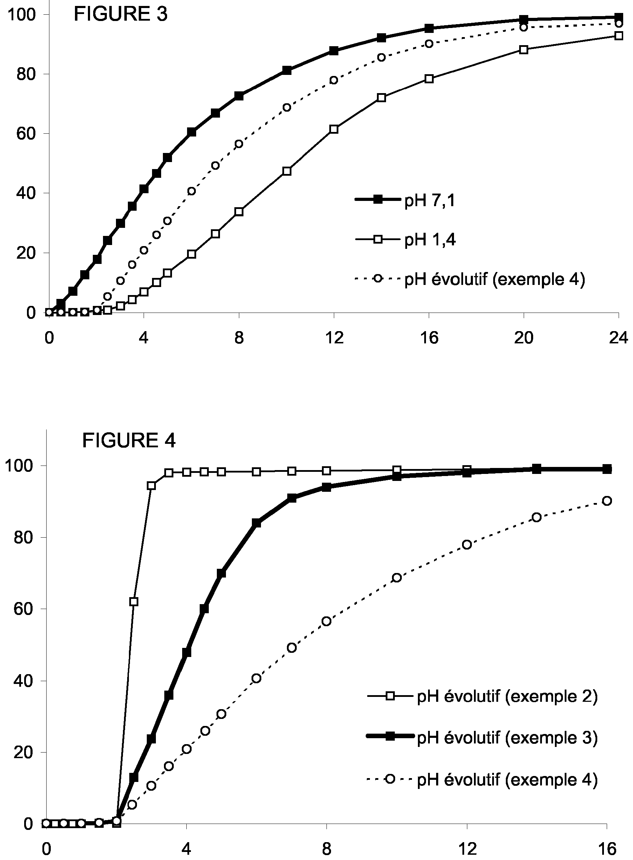

On a réalisé des tests de dissolutions sur les microparticules enrobées dans les milieux suivants : i) solution d'HCl à pH 1,4 ii) solution tamponnée KH₂PO₄/NaOH à pH 7,1 et iii) solution d'HCl à pH 1,4 2h puis en milieu tampon KH₂PO₄/NaOH à pH 7,1. Les résultats des tests de dissolution sont représentés en figure 3.

### EXEMPLE 5 : Préparation selon l'invention de microparticules enrobées conduisant à une libération retardée et prolongée de metformine, HCl.

On dissout à chaud 60,0g d'huile végétale hydrogénée Type 1 NF (Abitec) et 90,0g d'Acrycoat L100D (NP Pharm) dans 1350g d'isopropanol. La solution est pulvérisée sur 850g des microgranulés de metformine préparés dans l'exemple 1 dans un spray coater Glatt^{®} GPCG1. 455g des microparticules obtenues sont ensuite pelliculées avec 632g de la solution suivante : ethylcellulose 117g, povidone 66,3g, huile de ricin 11,7g, isopropanol 2242,5g. On obtient des microparticules enrobées.

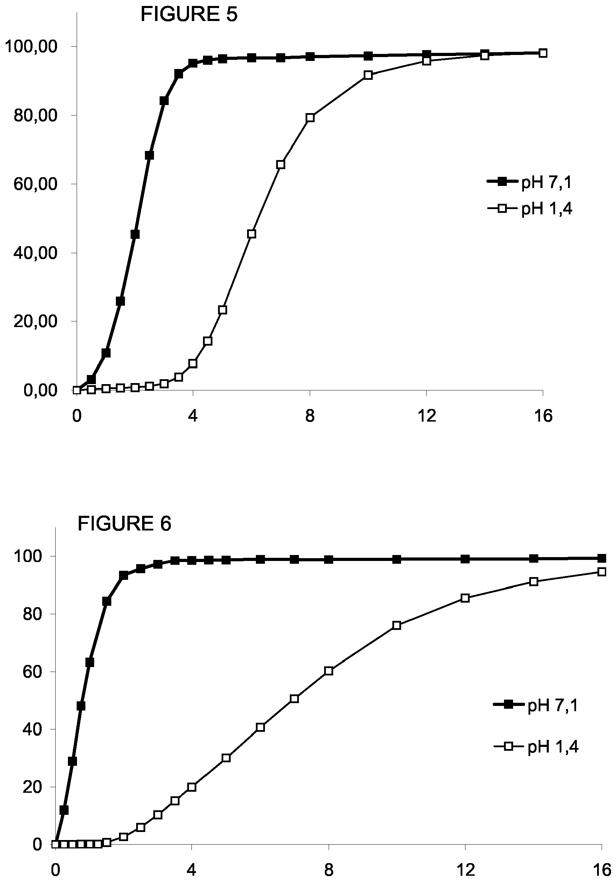

On a réalisé des tests de dissolutions sur les microparticules enrobées dans les milieux suivants : i) solution d'HCl à pH 1,4 ii) solution tamponnée KH₂PO₄/NaOH à pH 7,1. Les résultats des tests de dissolution sont représentés en figure 5.

### EXEMPLE 6 : Préparation selon l'invention de microparticules enrobées conduisant à une libération retardée et prolongée d'acyclovir.

On dissout 320,0g d'acyclovir et 80,0g de PVP dans un mélange contenant 668,6 g d'éthanol et 74,3g d'eau. La solution est pulvérisée sur 1600g de microsphères neutres (NP Pharm) dans un spray coater Glatt^{®} GPCG1.1.

On pellicule 500g de ces microgranulés avec la solution suivante : ethylcellulose 21,1g, PVP 8,3g, huile de ricin 2,5g, éthanol 606,4g.

On dissout à chaud 45,5g d'huile végétale hydrogénée Type 1 NF (Condea) et 68,3g de Kollicoat MAE 100P (BASF) dans 1023g d'éthanol. 867g de cette solution sont ensuite pulvérisés sur 455g des microparticules précédemment obtenues dans un spray coater Glatt^{®} GPCG1.1. On obtient des microparticules enrobées.

On a réalisé des tests de dissolutions sur les microparticules enrobées dans les milieux suivants : i) solution d'HCl à pH 1,4 ii) solution tamponnée KH₂PO₄/NaOH à pH 7,1. Les résultats des tests de dissolution sont représentés en figure 6.

Comme le montre la figure 4, dans les microparticules selon l'art antérieur, la libération du PA était immédiate dès que le pH de déclenchement de la libération était atteint. Par contre, avec les microparticules enrobées conformes à l'invention, lorsque le pH de déclenchement de la libération est atteint, la libération du PA est prolongée et contrôlée.

Comme le montrent les figures 2 à 5 et 6, les microparticules enrobées selon l'invention permettent effectivement de prolonger et de contrôler la libération d'un PA fortement soluble (metformine, HCl, figures 2 à 5) et d'un PA faiblement soluble (acyclovir, figure 6).

## Revendications

1. Microparticules "réservoir" enrobées contenant au moins un principe actif (PA) et étant du type de celles :
- constituées par des particules de PA recouvertes chacune d'au moins deux pellicules d'enrobage distinctes (A et B),
- et de diamètre moyen inférieur à 2000 microns ;
caractérisées en ce chacune d'elles comprend :
* au moins une pellicule d'enrobage (A) de composition suivante :
- entre 10 et 90 % en poids d'au moins un (co)polymère filmogène (A1) insoluble dans les liquides du tractus gastro-intestinal ;
- entre 5 et 50 % en poids d'au moins un (co)polymère (A2) soluble dans les liquides du tractus gastro-intestinal ;
- entre 1 et 30 % en poids d'au moins un plastifiant (A3) ;
- éventuellement entre 0 et 20 % en poids d'au moins un agent tensioactif et/ou lubrifiant(A4) ;
* et au moins une pellicule d'enrobage (B) de composition suivante :
- au moins un polymère (B1) hydrophile porteur de groupements ionisés à pH neutre
- et au moins un composé hydrophobe (B2) ;
- dans laquelle le ratio pondéral (B2)/(B1), est compris entre 0,2 et 1,5 ;
et en ce que, en combinaison, les pellicules d'enrobages (A et B) sont aptes :
- à assurer une libération du PA régie par deux mécanismes distincts de déclenchement, l'un basé sur une variation de pH et l'autre permettant la libération du PA, au bout d'un temps prédéterminé de résidence dans l'estomac,
- à induire un comportement de dissolution in vitro (réalisé dans un dissolutest à palette conforme à la Pharmacopée européenne 5.2 ou appareil de type II dans l'US Pharmacopeia 28-NF23, maintenu à 37 °C et agité à 100 tours/min) tel que :
- à pH constant 1,4, le profil de dissolution comporte une phase de latence de durée ajustable inférieure ou égale à 8 heures ;
- le passage de pH 1,4 à pH 7,1 conduit à une phase de libération prolongée de durée ajustable, débutant sans temps de latence et telle que t½ est compris entre 0,25 et 20 heures, où t½ est la durée nécessaire à la libération de 50% d'au moins l'un des principes actifs contenu dans les microparticles enrobées.

2. Microparticules selon la revendication 1, **caractérisées en ce que** le diamètre moyen desdites microparticules enrobées est compris entre 50 et 800 microns.

3. Microparticules selon la revendication 2, **caractérisées en ce que** le diamètre moyen desdites microparticules enrobées est compris entre 100 et 600 microns.

4. Microparticules selon la revendication 1, dans lesquelles la pellicule d'enrobage A a un taux de pelliculage (Tp_{A}) -exprimé en % en poids sec par rapport à la masse totale des microparticules- tel que 2% ≤ Tp_{A} ≤ 50%.

5. Microparticules selon l'une quelconque des revendications 1 à 4, dans lesquelles la pellicule d'enrobage B a un taux de pelliculage (Tp_{B}) -exprimé en % en poids sec par rapport à la masse totale des microparticules enrobées- inférieur ou égal à 50%.

6. Microparticules selon l'une quelconque des revendications 1 à 5, dans lesquelles les pellicules d'enrobages A et B représentent ensemble au plus 50 % en poids sec par rapport à la masse totale des microparticules enrobées.

7. Microparticules selon l'une quelconque des revendications 1 à 6, dans lesquelles :
* (A1) est choisi parmi :
• les dérivés non hydrosolubles de la cellulose,
• les dérivés acryliques,
• les polyvinylacétates,
• et leurs mélanges ;
* (A2) est choisi parmi :
• les (co)polymères azotés, sélectionnés dans le groupe comprenant les polyacrylamides, les poly-N-vinylamides, les polyvinylpyrrolidones (PVP) et les poly-N-vinyl-lactames ;
• les dérivés hydrosolubles de la cellulose,
• les alcools polyvinyliques (APV),
• les polyoxyéthylènes (POE),
• les polyéthylèneglycols (PEG)
• et leurs mélanges ;
* (A3) est choisi parmi :
• le glycérol et ses esters,
• les phtalates,
• les citrates,
• les sébaçates,
• les adipates,
• les azélates,
• les benzoates,
• les huiles végétales,
• les fumarates,
• les malates,
• les oxalates,
• les succinates,
• les butyrates,
• les esters de l'alcool cétylique,
• l'acide salicylique,
• la triacétine,
• les malonates,
• l'huile de ricin
• et leurs mélanges ;
* (A4) est choisi parmi :
• les tensioactifs anioniques, sélectionnés dans le sous-groupe des sels alcalins ou alcalinoterreux des acides gras,
• les tensioactifs non ioniques, sélectionnés dans le sous-groupe suivant :
o les huiles polyoxyéthylénées,
o les copolymères polyoxyéthylène-polyoxypropylène,
o les esters de sorbitan polyoxyéthylénés,
o les dérivés de l'huile de ricin polyoxyéthylénés,
o le stéarate de calcium, le stéarate de magnésium, le stéarate d'aluminium, le stéarate de zinc,
o les stéarylfumarates,
o les béhénates de glycérol,
o et leurs mélanges.

8. Microparticules selon la revendication 7, dans laquelle (A1) est choisi parmi l'éthylcellulose, l'acétate de cellulose, les copolymères d'acide (méth)acrylique et d'ester alkyle, les copolymères d'ester d'acide acrylique et méthacrylique porteur d'au moins un groupement ammonium quaternaire et leurs mélanges.

9. Microparticules selon l'une des revendications 1 à 8, dans lesquelles la pellicule d'enrobage A a la composition quantitative suivante en % en poids :
(A1) compris entre 15 et 80,
(A2) compris entre 10 et 40,
(A3) compris entre 2 et 20,
(A4) compris entre 0 et 15.

10. Microparticules selon l'une quelconque des revendications 1 à 9, dans lesquelles le polymère hydrophile porteur de groupements ionisés à pH neutre (B1) est choisi parmi :
∘ B1.a les copolymères d'acide (méth)acrylique et d'ester alkyle d'acide (méth)acrylique (EUDRAGIT ^{®} S ou L) ;
∘ B1.b les acétates de cellulose, les phtalates de cellulose, les succinates de celluloses, ;
∘ et leurs mélanges.

11. Microparticules selon l'une quelconque des revendications 1 à 10, dans lesquelles le composé (B2) est choisi parmi :
∘ B2.a cires prises à elles seules ou en mélanges entre-elles ;
∘ B2.b huiles végétales hydrogénées prises à elles seules ou en mélange entre-elles ;
∘ B2.c di et/ou tri esters du glycérol et d'au moins un acide gras ;
∘ B2.d
∘ et leurs mélanges.

12. Microparticules selon l'une quelconque des revendications 1 à 10, dans lesquelles le composé (B2) est choisi parmi: huile hydrogénée de graines de coton, huile hydrogénée de graines de soja, huile de palme hydrogénée, béhénate de glycérol, huile de ricin hydrogénée, tristéarine, tripalmitine, trimyristine, cire jaune, matière grasse dure ou matière grasse utile comme bases de suppositoires, matières grasses laitières anhydres, lanoline, palmitostéarate de glycerol, stéarate de glycérol, macrogolglycérides lauryliques, alcool cétylique, diisostéarate de polyglycérol, et leurs mélanges.

13. Microparticules selon la revendication 1, dans lesquelles le ratio pondéral (B2)/(B1), est compris entre 0,45 et 1,0.

14. Microparticules selon la revendication 1, dans lesquelles le composé hydrophobe (B2) est sélectionné parmi les produits cristallisés à l'état solide et ayant une température de fusion Tf(B2) ≥ 40°C.

15. Microparticules selon l'une quelconque des revendications précédentes, dans lesquelles le PA mis en oeuvre appartient à au moins l'une des familles de substances actives suivantes :
- les agents de traitement de l'abus d'alcool, tels que chlorazépate, chlordiazépoxyde, diazépam, disulfiram, hydroxyzine, naltrexone, et leurs sels, leurs esters, leurs hydrates, leurs polymorphes et leurs isomères ;
- les agents de traitement de la maladie d'Alzheimer, tels que CP 118954, donépezil, galanthamine, métrifonate, révastigmine, tacrine, TAK-147, et leurs sels, leurs esters, leurs hydrates, leurs polymorphes et leurs isomères ;
- les anesthésiques, tels que adrénaline, bupivacaïne, chloroprocaïne, desflurane, étidocaïne, levobupivacaïne, lidocaïne, midazolam, propofol, ropivacaïne, et leurs sels, leurs esters, leurs hydrates, leurs polymorphes et leurs isomères ;
- les agents de traitement de l'acromégalie, tels que octreotide, laureotide et pegvisomant, et leurs sels, leurs esters, leurs hydrates, leurs polymorphes et leurs isomères ;
- les analgésiques, tels que acétaminophène, aspirine, bupivacaïne, buprénorphine, butorphanol, célécoxib, clofénadol, choline, clonidine, codéine, diflunisal, dihydrocodéine, dihydroergotamine, dihydromorphine, éthylmorphine, étodolac, élétriptan, eptazocine, ergotamine, fentanyle, fénoprofène, acide hyaluronique, hydrocodone, hydromorphone, hylane, ibuprofène, indométhacine, ketorolac, kétotifène, levométhadone, levallorphane, lévorphanol, lidocaïne, acide méfénamique, méloxicam, mépéridine, méthadone, morphine, nabumétone, nalbuphine, néfopam, nalorphine, naloxone, naltrexone, naproxène, naratriptan, néfazodone, morméthadone, oxapozine, oxycodone, oxymorphone, pentazocine, péthidine, phenpyramide, piritramide, piroxicam, propoxyphène, réfécoxib, rizatriptan, kétoprofène, sulindac, sumatriptan, tébacone, tilidine, tolmétine, tramadol, zolmitriptan, et leurs sels, leurs esters, leurs hydrates, leurs polymorphes et leurs isomères ;
- les antiasthmatiques, tels que ablukast, azélastine, bunaprolast, cinalukast, cromitrile, cromolyne, énofélast, isambxole, kétotifène, levcromékaline, lodoxamide, montélukast, ontazolast, oxarbazole, oxatomide, piriprost potassium, pirolate, pobilukast, édamine, pranlukast, quazolast, répirinast, ritolukast, sulukast, tétrazolastméglumine, tiaramide, tibénélast, tomélukast, tranilast, verlukast, vérofylline, zarirlukast, et leurs sels, leurs esters, leurs hydrates, leurs polymorphes et leurs isomères
- les agents de traitement des allergies,
- les agents anticancéreux, tels que adriamycine, aldesleukine, allopurinol, altrétamine, amifostine, anastrozole, asparaginase, bétaméthasone, bexarotène, bicalutamide, bléomycine, busulfan, capécitabine, carboplatine, carmustine, chlorambucil, cisplatine, cladribine, œstrogène conjugué, cortisone, cyclophosphamide, cytarabine, dacarbazine, daunorubicine, dactinomycine, dénileukine, dexaméthasone, discodermolide, docétaxel, doxorubicine, éloposidem, épirubicine, époétine, épothilones, estramustine, œstrogène estérifié, éthinyl-œstradiol, étoposide, exemestane, flavopirdol, fluconazole, fludarabine, fluorouracile, flutamide, floxuridine, gemcitabine, gemtuzumab, goséréline, hexaméthylmélamine, hydrocortisone, hydroxyurée, idarubicine, ifosfamide, interféron, irinotécan, lémiposide, létrozole, leuprolide, lévamisole, lévothyroxine, lomustine, méchloréthamine, melphalan, mercaptopurine, mégestrol, méthotrexate, méthylprednisolone, méthyltestosterone, mithramycine, mitomycine, mitotane, mitoxantrone, mitozolomide, mutamycine, nilutamide, paclitaxel, pamidronate, pegaspargase, pentostatine, plicamycine, porfimer, prednisolone, procarbazine, rituximab, sargramostim, sémustine, streptozocine, tamoxifen, témozolamide, téniposide, testolactone, thioguanine, thiotépa, tomudex, topotécan, torémifène, trastumuzab, trétinoïne, sémustine, streptozolocine, valrubicine, verteprofine, vinblastine, vincristine, vindesine, vinorelbine, et leurs sels, leurs esters, leurs hydrates, leurs polymorphes et leurs isomères ;
- les anti-inflammatoires,
- les anticoagulants et antithrombotiques, tels que warfarine, daltéparine, héparine, tinzaparine, énoxaparine, danaparoïde, abciximab, alprostadil, altiplase, anagralide, anistreplase, argatroban, ataprost, bétaprost, camonagrel, cilostazol, clinprost, clopidogrel, cloricromène, dermatan, désirudine, domitroban, drotavérine, époprosténol, éptifibatide, fradafiban, gabexate, iloprost, isbogrel, lamifiban, lamotéplase, léfradafiban, lépirudin, lévosimendan, lexipafant, mélagatran, nafagrel, nafamostsat, nizofenone, orbifiban, ozagrel, pamicogrel, parnaparin, quinobendan, réteplase, sarpogralate, satigrel, silteplase, simendan, ticlopidine, vapiprost, tirofiban, xemilofiban, Y20811, et leurs sels, leurs esters, leurs hydrates, leurs polymorphes et leurs isomères ;
- les anti-convulsivants, tels que carbamazépine, clonazepam, clorazépine, diazépam, divalproex, éthosuximide, éthotion, felbamate, fosphénytoïne, gabapentine, lamotrigine, lévétiracétam, lorazépam, méphénytoïne, méphobarbital, métharbital, méthsuximide, oxcarbazépine, phénobarbital, phénytoïne, primidone, tiagabine, topiramate, acide valproïque, vigabatrine, zonisamide, et leurs sels, leurs esters, leurs hydrates, leurs polymorphes et leurs isomères ;
- les antiépileptiques,
- les antidiabétiques, tels que acarbose, acétohexamide, carbutamide, chlorpropamide, épalrestat, glibornuride, gliclazide, glimépiride, glipizide, gliquidone, glisoxepide, glyburide, glyhexamide, metformine, miglitol, natéglinide, orlistat, phénbutamide, pioglitazone, répaglinide, rosiglitazone, tolazamide, tolbutamide, tolcyclamide, tolrestat, troglitazone, voglibose, et leurs sels, leurs esters, leurs hydrates, leurs polymorphes et leurs isomères ;
- les antiémétiques, tels que alprazolam, benzquinamide, benztropine, bétahistine, chlorpromazine, dexaméthasone, difénidol, dimenhydrinate, diphénhydramine, dolasétron, dompéridone, dronabinol, dropéridol, granisétron, halopéridol, lorazépam, meclizine, méthylprednisolone, métoclopramide, ondansétron, perphénazine, prochlorpérazine, promethazine, scopolamine, tributine, triéthylpérazine, triflupromazine, triméthobenzamide, tropisétron, et leurs sels, leurs esters, leurs hydrates, leurs polymorphes et leurs isomères ;
- les antiglaucomes, tels que alprénoxime, dapiprazole, dipivéfrine, latanoprost, naboctate, pimabine, et leurs sels, leurs esters, leurs hydrates, leurs polymorphes et leurs isomères ;
- les antihistaminiques, tels que acépromazine, acrivastine, activastine, albutérol, alimémazine, antazoline, azélastine, bitoltérol, amlexanox, benzydamine, bromphéniramine, cétirizine, chlorphéniramine, cimétidine, cinnarizine, clémastine, clofédanol, cycloheptazine, cyproheptadine, diclofénac, difencloxazine, diphénhydramine, dotarizine, éphédrine, épinastine, épinéphrine, éthylnorépinéphrine, étybenzatropine, fenpentadiol, fenpotérol, fexofénadine, flurbiprofène, hydroxyzine, isoétharine, isoprotérénol, bromure d'ipratropium, kétorolac, lévocetirizine, lévomépromazine, loratidine, méquitazine, métaprotérénol, niaprazine, oxatomide, oxomémazine, phényléphrine, phénylpropanolamine, pirbutérol, prométhazine, pseudoéphédrine, pyrilamine, salmétérol, terbutaline, terfénadine, tranilast, dérivés de la xanthine, xylométazoline, et leurs sels, leurs esters, leurs hydrates, leurs polymorphes et leurs isomères ;
- les anti-infectieux, tels que les antibiotiques, les antifongiques, les antiviraux, tels que abacavir, acyclovir, albendazole, amantadine, amphotéricine, amikacine, acide aminosalicylique, amoxycilline, ampicilline, amprénavir, atovaquine, azithromycine, aztréonam, carbenicilline, céfaclor, céfadroxil, céfamandole, céfazolin, cefdinir, céfépime, céfexime, céfopérazone, céfotaxime, céfotitam, céfopérazone, céfoxitine, cefpodoxine, cefprozil, ceftazidime, ceftibutène, ceftizoxime, ceftriaxone, céfuroxime, céphalexine, chloroquine, cidofovir, cilastatine, ciprofloxacine, clarithromycine, acide clavulanique, clindamycine, colistiméthate, dalfopristine, dapsone, daunorubicine, delavirdine, déméclocycline, didanosine, doxycycline, doxorubicine, éfavirenz, énoxacine, érythromycine, éthambutol, éthionamide, famcyclovir, fluconazole, flucytocine, foscarnet, fosfomycine, ganciclovir, gatifloxacine, griseofulvine, hydroxychloroquine, imipenem, indinavir, interféron, isoniazide, itraconazole, ivermectil, kétoconazole, lamivudine, lévofloxacine, linizolide, loméfloxacine, loracarbef, mébendazole, méfloquine, méropénem, méthanamine, métronidazole, minocycline, moxefloxacine, acide naldixique, nelfinavir, néomycine, névirapine, nitorfurantoïne, norfloxacine, ofloxacine, oseltamivir, oxytetracycline, palivizumab, pénicilline, perfloxacine, pipéracilline, praziquantel, pyrazinamide, pyriméthamine, quinidine, quinupristine, rétonavir, ribavirine, rifabutine, rifampicine, rimantadine, saquinavir, sparfloxacine, stavudine, streptomycine, sulfamethoxazole, tétramycine, terbinafine, tétracycline, ticarcillin, thiabendazole, tobramycine, triméthoprim, trimétraxate, troléandomycine, trovafloxacine, valacyclovir, vancomycine, zalcitabine, zanamivir, zidovudine, et leurs sels, leurs esters, leurs hydrates, leurs polymorphes et leurs isomères ;
- les antiparkinsoniens, tels que amantadine, adrogolide, altinicline, benzatropine, bipéridène, brasofensine, bromocriptine, budipine, cabergoline, CHF-1301, dihydrexidine, entacapone, etilevodopa, idazoxane, iométopane, lazabémide, melevodopa, carbidopa, levodopa, mofégiline, moxiraprine, pergolide, pramipexole, quinélorane, rasagiline, ropinirole, séligiline, talipexole, tolcapone, trihexyphenidyle, et leurs sels, leurs esters, leurs hydrates, leurs polymorphes et leurs isomères ;
- les agents antirhumatismants, tels que azathiprine, bétaméthasone, célécoxib, cyclosporine, diclofénac, hydroxychloroquine, indométhacine, infliximab, acide mercaptobutanedioïque, méthylprednisolone, naproxène, pénicillamine, piroxicam, prednisolone, sulfasalazine, et leurs sels, leurs esters, leurs hydrates, leurs polymorphes et leurs isomères ;
- les anti-agrégeants plaquettaires, tels que abciximab, anagrélide, aspirine, cilostazol, clopidogrel, dipyridamole, époprosténol, éptifibatide, ticlopidine, tinofiban, et leurs sels, leurs esters, leurs hydrates, leurs polymorphes et leurs isomères ;
- les antispasmodiques et les anticholinergiques, tels que aspirine, atropine, diclofénac, hyoscyamine, mésoprostol, méthocarbamol, phénobarbital, scopolamine, et leurs sels, leurs esters, leurs hydrates, leurs polymorphes et leurs isomères ;
- les antitussifs, tels que acétaminophène, acrivastine, albutérol, benzonatate, béractant, bromphéniramine, caféine, calfactant, carbétapentane, chlorphéniramine, codéine, colfuscérine, dextromethorpham, dornase alpha, doxylamine, épinephrine, fexofénadine, guaïfénésine, ipratropium, lévalbutérol, métaprotérénol, montélukast, pentoxyphilline, phényléphrine, phénylpropanolamine, pirbutérol, poractant alpha, pseudoéphédrine, pyrilamine, salbuterol, salmeterol, terbutaline, theophylline, zafirlukast, zileuton et leur sels, leurs esters, leurs hydrates, leurs polymorphes et leurs isomères ;
- les inhibiteurs de l'anhydrase carbonique, tels que acétazolamide, dichlorphénamide, dorzolamide, méthazolamide, sézolamide, et leurs sels, leurs esters, leurs hydrates, leurs polymorphes et leurs isomères ;
- les agents cardiovasculaires, tels que les hypolipémiants, les anti-arythmiques, les vasodilatateurs, les antiangineux, les anti-hypertenseurs, les vasoprotecteurs, tels que abciximab, acébutolol, activase, adénosine, adrénaline, amidarone, amiloride, amlodipine, nitrate d'amyle, aténolol, atorvastatine, benzépril, bépiridil, bétaxalol, bisoprolol, candésartan, captopril, carténolol, carvédilol, cérivastatine, chlorthalidone, chlorthiazole, clofibrate, clonidine, colestipol, colosévélam, digoxine, diltiazem, disopyramide, dobutamine, dofétilide, doxazosine, énalapril, époprosténol, éprosartan, esmolol, éthacrynate, érythrityl, félodipine, fénoidapam, fosinopril, flécaïnide, flurosémide, fluvastatine, gemfibrozil, hydrochlorthiazide, hydroflumethazine, ibutilide, indapamide, isosorbide, irbésartan, labétolol, lacidipine, lisinopril, losartan, lovastatine, mécamylamine, métoprolol, métarminol, métazolone, méthylchlothiazide, méthyldopa, métyrosine, mexilétine, midrodine, milrinone, moexipril, nadolol, niacine, nicardipine, nicorandil, nifédipine, nimodipine, nisoldipine, nitroglycérine, phénoxybenzamine, périndopril, polythiazide, pravastatine, prazosine, procaïnamide, propafénone, propranolol, quanfacine, quinapril, quinidine, ranipril, rétéplase, simvastatine, sotalol, spironolactone, streptokinase, telmisartan, térazosine, timolol, tocaïnamide, torsémide, trandolapril, triamtérène, trapidil, valsartan, et leurs sels, leurs esters, leurs hydrates, leurs polymorphes et leurs isomères ;
- les vasodilatateurs, tels que adénosine, alvérine, caféine, dihydroergocornine, énalapril, énoximone, iloprost, kalléone, lidoflazine, nicardipine, nimodipine, acide nicotinique, papaverine, pilocarpine, salbutamol, théophylline, trandolapril, uradipil, vincamine, et leurs sels, leurs esters, leurs hydrates, leurs polymorphes et leurs isomères ;
- les inhibiteurs de cholinestérase, tels que donépezil, édrophonium, néostigmine, pyridostigmine, rivastigmine, tacrine, et leurs sels, leurs esters, leurs hydrates, leurs polymorphes et leurs isomères ;
- les agents de traitement des désordres du système nerveux central,
- les stimulants du système nerveux central, tels que caféine, doxapram, dexoamphétamine, donépézil, édorphonium, méthamphétamine, méthylphénidate, modafinil, néostigmine, pémoline, phentermine, pyridostigmine, rivastigmine, tacrine et leur sels, leurs esters, leurs hydrates, leurs polymorphes et leurs isomères ;
- les contraceptifs, tels que désogestral, éthinyl-œstradiol, éthynodiol, lévonorgestrel, médroxyprogestérone, mestranol, norgestimate, noréthindrone, norgestrel, et leurs sels, leurs esters, leurs hydrates, leurs polymorphes et leurs isomères ;
- les promoteurs de fécondité,
- les inducteurs et inhibiteurs du travail utérin,
- les agents de traitement de la mucoviscidose, tels que domase alpha, pancrélipase, tobramycine, et leurs sels, leurs esters, leurs hydrates, leurs polymorphes et leurs isomères ;
- les agonistes des récepteurs de la dopamine, tels que amantadine, cabergoline, fenoldopam, pergolide, pramipezal, ropinirole et leur sels, leurs esters, leurs hydrates, leurs polymorphes et leurs isomères ;
- les agents de traitement de l'endométriose, tels que danazol, goséréline, leuprolide, nafaréline, norethindrone, et leurs sels, leurs esters, leurs hydrates, leurs polymorphes et leurs isomères ;
- les agents de traitement des dysfonctionnements érectiles, tels que alprostadil, sildénafil, yohimbine, et leurs sels, leurs esters, leurs hydrates, leurs polymorphes et leurs isomères ;
- les agents de traitement de la fertilité, tels que citrorélix, clomiphène, follitropine, ganirélix, gonadotropine, ménotropine, progésterone, urofollitropine, et leurs sels, leurs esters, leurs hydrates, leurs polymorphes et leurs isomères ;
- les agents de traitements des troubles gastro-intestinaux, tels que alosétron, bisacodyl, subsalicylate de bismuth, célécoxib, cimétidine, difoxine, diphéoxylate, docusate, ésoméprazole, famotidine, glycopyrrolate, infliximab, lansoprazole, lopéramide, métaclopramide, nizatidine, oméprazole, pantoprazole, rabéprazole, ranitidine, siméthicone, sucralfate, et leurs sels, leurs esters, leurs hydrates, leurs polymorphes et leurs isomères ;
- les immunomodulateurs et les immunosuppresseurs, tels que azathioprine, ceftizoxine, cyclosporine, daclizumab, glatiramer, immunoglobuline, interféron, leflunomide, lévamisol, mycophénolate, phthalidomide, ribavirine, sirolimus, et leurs sels, leurs esters, leurs hydrates, leurs polymorphes et leurs isomères ;
- les agents de traitement des troubles de la mémoire,
- les antimigraineux, tels que acétaminophène, dihydroergotamine, divalproex, ergotamine, propranolol, risatriptan, sumatriptan, trimetrexate, et leurs sels, leurs esters, leurs hydrates, leurs polymorphes et leurs isomères ;
- les relaxants des muscles, tels que chlorure d'alcuronium, azapropazone, atracurium, baclofène, carisoprodol, dérivés de quinine, chloromézanone, chlorophénésincarbamate, chlorozoxazone, cyclobenzaprine, dantrolène, bromure de décaméthonium, chlorure de dimethyltubocurarinium, doxacurium, fényramidol, triethiodure de gallamine, guaïfénésine, bromure d'hexafluorenium, bromure d'hexacarbacholine, mémantin, méphénésine, méprobamate, métamisol, métaxalone, méthocarbamol, mivacurium, orphénadrine, pancuronium, phénazone, phénprobamate, pipécuronium, rapacuronium, rocuronium, succinylcholine, chlorure de suxaméthonium, tétrazépam, tizanidine, chlorure de tubocurarine, tybamate, vecuronium, et leurs sels, leurs esters, leurs hydrates, leurs polymorphes et leurs isomères ;
- les analogues de nucléosides, tels que abacavir, acyclovir, didanosine, gamciclovir, gemcitabine, lamivudine, ribavirine, stavudine, zalcitabine, et leurs sels, leurs esters, leurs hydrates, leurs polymorphes et leurs isomères ;
- les agents de traitement de l'ostéoporose, tels que alendronate, calcitonine, œstradiol, estropipate, médroxyprogestérone, noréthindrone, norgestimate, pamidronate, raloxifène, risdronate, zolédronate, et leurs sels, leurs esters, leurs hydrates, leurs polymorphes et leurs isomères ;
- les parasympathomimétiques, tels que béthanechol, bipéridine, édrophonium, glycopyrolate, hyoscyamine, pilocarpine, tacrine, yohimbine, et leurs sels, leurs esters, leurs hydrates, leurs polymorphes et leurs isomères ;
- les prostaglandines, tels que alprostadil, époprosténol, misoprostol, et leurs sels, leurs esters, leurs hydrates, leurs polymorphes et leurs isomères ;
- les agents psychothérapeutiques, tels que acétophénazine, alentémol, alpertine, alprazolam, amitriptyline, apriprazole, azapérone, batélapine, béfipiride, benpéridol, benzindopyrine, bimithil, biripérone, brofoxine, brompéridol, bronipéridol, bupropione, buspirone, butaclamol, butapérazine, butapérazin, carphénazine, carvotroline, cériclamine, chlorazépine, chlordiazépoxide, chlorpromazine, chlorprothixène, cinpérène, cintriamide, citalopram, clomacrane, clonazépam, clopenthixol, clopimozide, clopipazane, cloropérone, clothiapine, clothixamide, clozapine, cyclophénazine, dapiprazole, dapoxétine, désipramine, divalproex, dipyridamole, doxépine, dropéridol, duloxétine, eltoprazine, eptipirone, étazolate, fénimide, flibansérine, flucindole, flumézapine, fluoxétine, fluphénazine, fluspipérone, fluspirilène, flutroline, fluvoxamine, gépirone, gévotroline, halopémide, halopéridol, hydroxyzine, hydroxynortriptyline, ilopéridone, imidoline, lamotrigine, loxapine, enpérone, mazapertine, méphobarbital, méprobamate, mésoridazine, mésoridazine, milnacipran, mirtazépine, métiapine, milenpérone, milipertine, molindone, nafadotride, naranol, nefazodone, neflumozide, ocapéridone, odapipam, olanzapine, oxethiazine, oxipéromide, pagoclone, palipéridone, paroxitène, penfluridol, pentiapine, perphénazine, phénelzine, pimozide, pinoxépine, pipampérone, pipéracétazine, pipotiazine, piquindone, pirlindole, pivagabine, pramipexole, prochlorpérazine, promazine, quétiapine, réboxetine, rémoxipride, rispéridone, rimcazole, robolzotan, sélégiline, sépéridol, sertraline, sertindole, seteptiline, sétopérone, spipérone, sunipitrone, tépirindole, thioridazine, thiothixène, tiapride, tiopéridone, tiospirone, topiramate, tranylcypromine, trifluopérazine, triflupéridol, triflupromazine, trimipramine, venlafaxine, ziprasidone, et leurs sels, leurs esters, leurs hydrates, leurs polymorphes et leurs isomères ;
- les sédatifs, les hypnotiques et tranquillisants, tels que bromazépam, buspirone, clazolam, clobazam, chlorazépate, diazépam, démoxépam, dexmédétomidine, diphényhydramine, doxylamine, enciprazine, estrazolam, hydroxyzine, ketazolam, lorazatone, lorazépam, loxapine, médazépam, mépéridine, méthobarbital, midazolam, nabilone, nisobamate, oxazépam, pentobarbital, prométhazine, propofol, triazolam, zaléplon, zolpidem, et leurs sels, leurs esters, leurs hydrates, leurs polymorphes et leurs isomères ;
- les neuroleptiques,
- les anxiolytiques,
- les psychostimulants,
- les antidépresseurs,
- les agents de traitements dermatologiques, tels que acitrétine, alclométasone, alitretinoïne, bétaméthasone, calciprotrine, chlorhexidine, clobétasol, clocortolone, clotriamozole, collagénase, cyclosporine, désonide, difluorosone, doxépine, eflomithine, finastéride, fluocinolone, flurandrénolide, fluticasone, halobétasol, hydrochloroquine, hydroquinone, hydroxyzine, kétoconazole, mafénide, malathion, ménobenzone, néostigmine, nystatine, podofilox, povidone, tazorotène, trétinoïne, et leurs sels, leurs esters, leurs hydrates, leurs polymorphes et leurs isomères ;
- les stéroïdes et les hormones, tels que alclométasone, bétaméthasone, calcitonine, citrorélix, clobétasol, clocortolone, les cortisones, danazol, desmopressine, désonide, désogestrel, désoximétasone, dexaméthasone, diflorasone, œstradiol, œstrogènes, estropipate, éthynilestradiol, fluocinolone, flurandrénolide, fluticasone, glucagon, gonadotropine, goséréline, halobétasol, hydrocortisone, leuprolide, lévonorgestrel, lévothyroxine, médroxyprogestérone, les ménotropines, méthylprednisolone, méthyltestosterone, mométasone, naféréline, norditropine, noréthindrone, norgestrel, octréolide, oxandrolone, oxymétholone, polytropine, prednicarbate, prednisolone, progestérone, sermoréline, somatropine, stanozolol, testostérone, urofollitropine, et leurs sels, leurs esters, leurs hydrates, leurs polymorphes et leurs isomères.

16. Formulation pharmaceutique, vétérinaire ou diététique **caractérisée en ce qu'**elle comprend une pluralité de microparticules enrobées telles que définies dans l'une quelconque des revendications 1 à 15, avantageusement au moins 500 microparticules, et **en ce qu'**elle comprend en outre au moins un principe actif sous une forme à libération immédiate.

17. Formulation selon la revendication 16, comprenant une pluralité de populations de microparticules, lesdites populations se distinguant les unes des autres par leur temps de latence et/ou par leur pH de déclenchement et/ou par leur vitesse de libération et/ou par le principe actif qu'elles contiennent.

18. Utilisation des microparticules enrobées telles que définies dans l'une quelconque des revendications 1 à 15, pour la préparation de formulations pharmaceutiques, vétérinaires ou diététiques telles que définies dans l'une quelconque des revendications 16 et 17.

## Patentansprüche

1. Beschichtete "Reservoir"-Mikropartikel, die mindestens einen Wirkstoff (PA) enthalten und vom Typ derjenigen sind, die:
- aus PA-Partikeln bestehen, die jeweils mit mindestens zwei verschiedenen Beschichtungsfilmen (A und B) überzogen sind,
- und einen mittleren Durchmesser von weniger als 2000 Mikrometern aufweisen;
**dadurch gekennzeichnet, dass** jeder von ihnen umfasst:
* mindestens einen Beschichtungsfilm (A) mit folgender Zusammensetzung:
- zwischen 10 und 90 Gew.-% mindestens eines filmbildenden (Co-) Polymers (A1), das in den Flüssigkeiten des Magen-Darm-Trakts unlöslich ist;
- zwischen 5 und 50 Gew.-% mindestens eines (Co-)Polymers (A2), das in den Flüssigkeiten des Magen-Darm-Trakts löslich ist;
- zwischen 1 und 30 Gew.-% mindestens eines Weichmachers (A3);
- gegebenenfalls zwischen 0 und 20 Gew.-% mindestens eines Tensids und/oder Gleitmittels (A4);
* und mindestens einen Beschichtungsfilm (B) mit folgender Zusammensetzung:
- mindestens ein hydrophiles Polymer (B1), das bei neutralem pH-Wert ionisierte Gruppen trägt
- und mindestens eine hydrophobe Verbindung (B2);
- wobei das Gewichtsverhältnis (B2)/(B1) im Bereich von einschließlich 0,2 bis 1,5 liegt;
und dadurch, dass die Beschichtungsfilme (A und B) in Kombination geeignet sind:
- eine Freisetzung des PA zu gewährleisten, die durch zwei verschiedene Auslösemechanismen gesteuert wird, von denen der eine auf einer pH-Wert-Änderung beruht und der andere die Freisetzung des PA nach einer vorbestimmten Verweilzeit im Magen ermöglicht,
- in vitro ein Auflösungsverhalten zu induzieren (durchgeführt in einem Paletten-Dissolutest gemäß der Europäischen Pharmacopeia 5.2 oder in einem Gerät vom Typ II in der US Pharmacopeia 28-NF23, das bei 37°C gehalten und bei 100 U/min geschüttelt wird), wie z.B.:
- bei einem konstanten pH-Wert von 1,4 umfasst das Auflösungsprofil eine Latenzphase mit einer einstellbaren Dauer von 8 Stunden oder weniger;
- der Übergang von pH 1,4 auf pH 7,1 führt zu einer verlängerten Freisetzungsphase mit einstellbarer Dauer, die ohne Latenzzeit beginnt und so beschaffen ist, dass t½ zwischen einschließlich 0,25 und 20 Stunden liegt, wobei t½ die Zeit ist, die für die Freisetzung von 50 % mindestens eines der in den beschichteten Mikropartikeln enthaltenen Wirkstoffe benötigt wird.

2. Mikropartikel nach Anspruch 1, **dadurch gekennzeichnet, dass** der mittlere Durchmesser der beschichteten Mikropartikel zwischen einschließlich 50 und 800 Mikrometern liegt.

3. Mikropartikel nach Anspruch 2, **dadurch gekennzeichnet, dass** der mittlere Durchmesser der beschichteten Mikropartikel zwischen einschließlich 100 und 600 Mikrometern liegt.

4. Mikropartikel nach Anspruch 1, wobei der Beschichtungsfilm A einen Beschichtungsgrad (Tp_{A}) - ausgedrückt in % Trockengewicht bezogen auf die Gesamtmasse der Mikropartikel - von 2% ≤ TpA ≤ 50% aufweist.

5. Mikropartikel nach einem der Ansprüche 1 bis 4, wobei der Beschichtungsfilm B einen Beschichtungsgrad (Tp_{B}) - ausgedrückt in % Trockengewicht bezogen auf die Gesamtmasse der beschichteten Mikropartikel - von 50 % oder weniger aufweist.

6. Mikropartikel nach einem der Ansprüche 1 bis 5, wobei die Beschichtungsfilme A und B zusammen höchstens 50 % Trockengewicht, bezogen auf die Gesamtmasse der beschichteten Mikropartikel, ausmachen.

7. Mikropartikel nach einem der Ansprüche 1 bis 6, wobei:
* (A1) ausgewählt ist aus:
• nicht wasserlöslichen Cellulosederivaten,
• Acrylderivaten,
• Polyvinylacetaten,
• und deren Mischungen;
* (A2) ausgewählt ist aus:
• stickstoffhaltigen (Co-)Polymeren, ausgewählt aus der Gruppe umfassend Polyacrylamide, Poly-N-vinylamide, Polyvinylpyrrolidone (PVP) und Poly-N-vinyllactame;
• wasserlöslichen Cellulosederivaten,
• Polyvinylalkoholen (PVA),
• Polyoxyethylenen (POE),
• Polyethylenglykolen (PEG)
• und deren Mischungen;
(A3) ausgewählt ist aus:
• Glycerin und seinen Estern,
• Phthalaten,
• Citraten,
• Sebazaten,
• Adipaten,
• Azelaten,
• Benzoaten,
• pflanzlichen Ölen,
• Fumaraten,
• Malaten,
• Oxalaten,
• Succinaten,
• Butyraten,
• Estern des Cetylalkohols,
• Salicylsäure,
• Triacetin,
• Malonaten,
• Rizinusöl
• und deren Mischungen;
(A4) ausgewählt ist aus:
• anionischen Tensiden, ausgewählt aus der Untergruppe der Alkali- oder Erdalkalisalze von Fettsäuren,
• nicht-ionischen Tensiden, ausgewählt aus der folgenden Untergruppe:
o polyoxyethylenierte Öle,
o Polyoxyethylen-Polyoxypropylen-Copolymere,
o polyoxyethylenierte Sorbitanester,
o polyoxyethylenierte Rizinusölderivate,
∘ Calciumstearat, Magnesiumstearat, Aluminiumstearat, Zinkstearat,
∘ Stearylfumarate,
∘ Glycerinbehenate
∘ und deren Mischungen.

8. Mikropartikel nach Anspruch 7, wobei (A1) ausgewählt ist aus Ethylcellulose, Celluloseacetat, Copolymeren von (Meth)acrylsäure und Alkylester, Copolymeren von Acryl- und Methacrylsäureestern, die mindestens eine quarternäre Ammoniumgruppe tragen, und deren Mischungen.

9. Mikropartikel nach einem der Ansprüche 1 bis 8, wobei der Beschichtungsfilm A die folgende quantitative Zusammensetzung in Gew.-% aufweist:
(A1) zwischen einschließlich 15 und 80,
(A2) zwischen einschließlich 10 und 40,
(A3) zwischen einschließlich 2 und 20,
(A4) zwischen einschließlich 0 und 15.

10. Mikropartikel nach einem der Ansprüche 1 bis 9, wobei das hydrophile Polymer, das bei neutralem pH-Wert ionisierte Gruppen trägt (B1), ausgewählt ist aus:
o B1.a Copolymere von (Meth)acrylsäure und (Meth)acrylsäurealkylester (EUDRAGIT ^{®} S oder L) ;
o B1.b Celluloseacetate, Cellulosephthalate, Cellulosesuccinate;
o und deren Mischungen.

11. Mikropartikel nach einem der Ansprüche 1 bis 10, wobei die Verbindung (B2) ausgewählt ist aus :
o B2.a Wachse, für sich genommen oder im Gemisch miteinander;
o B2.b hydrierte pflanzliche Öle, für sich genommen oder im Gemisch miteinander;
o B2.c Di- und/oder Tri-Ester von Glycerin und mindestens einer Fettsäure ;
o B2.d
und deren Mischungen.

12. Mikropartikel nach einem der Ansprüche 1 bis 10, wobei die Verbindung (B2) ausgewählt ist aus: Hydriertes Baumwollsamenöl, hydriertes Sojabohnenöl, hydriertes Palmöl, Glycerolbehenat, hydriertes Rizinusöl, Tristearin, Tripalmitin, Trimyristin, gelbes Wachs, Hartfett oder als Zäpfchengrundlage verwendbares Fett, wasserfreie Milchfette, Lanolin, Glycerinpalmitostearat, Glycerinstearat, Laurylmakrogolglyceride, Cetylalkohol, Polyglycerindiisostearat und deren Mischungen.

13. Mikropartikel nach Anspruch 1, wobei das Gewichtsverhältnis (B2)/(B1) im Bereich von einschließlich 0,45 bis 1,0 liegt.

14. Mikropartikel nach Anspruch 1, wobei die hydrophobe Verbindung (B2) ausgewählt ist aus Produkten, die im festen Zustand kristallisiert sind und eine Schmelztemperatur Tf(B2) ≥ 40°C aufweisen.

15. Mikropartikel nach einem der vorhergehenden Ansprüche, wobei der eingesetzte PA zu mindestens einer der folgenden Wirkstofffamilien gehört:
- Mittel zur Behandlung von Alkoholmissbrauch, wie Chlorazepat, Chlordiazepoxid, Diazepam, Disulfiram, Hydroxyzin, Naltrexon, sowie deren Salze, Ester, Hydrate, Polymorphe und Isomere;
- Mittel zur Behandlung der Alzheimer-Krankheit, wie CP 118954, Donepezil, Galanthamin, Metrifonat, Revastigmin, Tacrin, TAK-147, sowie deren Salze, Ester, Hydrate, Polymorphe und Isomere;
- Anästhetika, wie Adrenalin, Bupivacain, Chloroprocain, Desfluran, Etidocain, Levobupivacain, Lidocain, Midazolam, Propofol, Ropivacain, sowie deren Salze, Ester, Hydrate, Polymorphe und Isomere;
- Mittel zur Behandlung von Akromegalie, wie Octreotid, Laureotid und Pegvisomant, sowie deren Salze, Ester, Hydrate, Polymorphe und Isomere;
- Analgetika, wie Acetaminophen, Aspirin, Bupivacain, Buprenorphin, Butorphanol, Celecoxib, Clofenadol, Cholin, Clonidin, Codein, Diflunisal, Dihydrocodein, Dihydroergotamin, Dihydromorphin, Ethylmorphin, Etodolac, Eletriptan, Eptazocin, Ergotamin, Fentanyl, Fenoprofen, Hyaluronsäure, Hydrocodon, Hydromorphon, Hylane, Ibuprofen, Indomethacin, Ketorolac, Ketotifen, Levomethadon, Levallorphan, Levorphanol, Lidocain, Mefenaminsäure, Meloxicam, Meperidin, Methadon, Morphin, Nabumeton, Nalbuphin, Nefopam, Nalorphin, Naloxon, Naltrexon, Naproxen, Naratriptan, Nefazodon, Mormethadon, Oxapozin, Oxycodon, Oxymorphon, Pentazocin, Pethidin, Phenpyramid, Piritramid, Piroxicam, Propoxyphen, Refoxib, Rizatriptan, Ketoprofen, Sulindac, Sumatriptan, Tebacon, Tilidin, Tolmetin, Tramadol, Zolmitriptan, sowie deren Salze, Ester, Hydrate, Polymorphe und Isomere;
- Antiasthmatika, wie Ablukast, Azelastin, Bunaprolast, Cinalukast, Cromitril, Cromolyn, Enofelast, Isambxol, Ketotifen, Levcromekalin, Lodoxamid, Montelukast, Ontazolast, Oxarbazol, Oxatomid, Piriprost-Kalium, Pirolat, Pobilukast, Edamin, Pranlukast, Quazolast, Repirinast, Ritolukast, Sulukast, Tetrazolastmeglumin, Tiaramid, Tibenelast, Tomelukast, Tranilast, Verlukast, Verofyllin, Zarirlukast, sowie deren Salze, Ester, Hydrate, Polymorphe und Isomere.
- Mittel zur Behandlung von Allergien,
- Mittel gegen Krebs, wie Adriamycin, Aldesleukin, Allopurinol, Altretamin, Amifostin, Anastrozol, Asparaginase, Betamethason, Bexaroten, Bicalutamid, Bleomycin, Busulfan, Capecitabin, Carboplatin, Carmustin, Chlorambucil, Cisplatin, Cladribin, konjugiertes Östrogen, Cortison, Cyclophosphamid, Cytarabin, Dacarbazin, Daunorubicin, Dactinomycin, Denileukin, Dexamethason, Discodermolid, Docetaxel, Doxorubicin, Eloposidem, Epirubicin, Epoetin, Epothilone, Estramustin, verestertes Östrogen, Ethinyl-Östradiol, Etoposid, Exemestan, Flavopirdol, Fluconazol, Fludarabin, Fluoruracil, Flutamid, Floxuridin, Gemcitabin, Gemtuzumab, Goserelin, Hexamethylmelamin, Hydrocortison, Hydroxyurea, Idarubicin, Ifosfamid, Interferon, Irinotecan, Lemiposid, Letrozol, Leuprolid, Levamisol, Levothyroxin, Lomustin, Mechlorethamin, Melphalan, Mercaptopurin, Megestrol, Methotrexat, Methylprednisolon, Methyltestosteron, Mithramycin, Mitomycin, Mitotan, Mitoxantron, Mitozolomid, Mutamycin, Nilutamid, Paclitaxel, Pamidronat, Pegaspargase, Pentostatin, Plicamycin, Porfimer, Prednisolon, Procarbazin, Rituximab, Sargramostim, Semustin, Streptozocin, Tamoxifen, Temozolamid, Teniposid, Testolacton, Thioguanin, Thiotepa, Tomudex, Topotecan, Toremifen, Trastumuzab, Tretinoin, Semustin, Streptozolocin, Valrubicin, Verteprofin, Vinblastin, Vincristin, Vindesin, Vinorelbin, sowie deren Salze, Ester, Hydrate, Polymorphe und Isomere;
- entzündungshemmende Mittel,
- Antikoagulantien und Antithrombotika, wie Warfarin, Dalteparin, Heparin, Tinzaparin, Enoxaparin, Danaparoid, Abciximab, Alprostadil, Altiplase, Anagralide, Anistreplase, Argatroban, Ataprost, Betaprost, Camonagrel, Cilostazol, Clinprost, Clopidogrel, Cloricromen, Dermatan, Desirudin, Domitroban, Drotaverin, Epoprostenol, Eptifibatid, Fradafiban, Gabexat, Iloprost, Isbogrel, Lamifiban, Lamoteplase, Lefradafiban, Lepirudin, Levosimendan, Lexipafant, Melagatran, Nafagrel, Nafamostsat, Nizofenon, Orbifiban, Ozagrel, Pamicogrel, Parnaparin, Quinobendan, Reteplase, Sarpogralat, Satigrel, Silteplase, Simendan, Ticlopidin, Vapiprost, Tirofiban, Xemilofiban, Y20811, sowie deren Salze, Ester, Hydrate, Polymorphe und Isomere;
- Antikonvulsiva, wie Carbamazepin, Clonazepam, Clorazepin, Diazepam, Divalproex, Ethosuximid, Ethotion, Felbamat, Fosphenytoin, Gabapentin, Lamotrigin, Levetiracetam, Lorazepam, Mephenytoin, Mephobarbital, Metharbital, Methsuximid, Oxcarbazepin, Phenobarbital, Phenytoin, Primidon, Tiagabin, Topiramat, Valproinsäure, Vigabatrin, Zonisamid, sowie deren Salze, Ester, Hydrate, Polymorphe und Isomere;
- Antiepileptika,
- Antidiabetika, wie Acarbose, Acetohexamid, Carbutamid, Chlorpropamid, Epalrestat, Glibornurid, Gliclazid, Glimepirid, Glipizid, Gliquidon, Glisoxepid, Glyburid, Glyhexamid, Metformin, Miglitol, Nateglinid, Orlistat, Phenbutamid, Pioglitazon, Repaglinid, Rosiglitazon, Tolazamid, Tolbutamid, Tolcyclamid, Tolrestat, Troglitazon, Voglibose, sowie deren Salze, Ester, Hydrate, Polymorphe und Isomere;
- Antiemetika wie Alprazolam, Benzquinamid, Benztropin, Betahistin, Chlorpromazin, Dexamethason, Difenidol, Dimenhydrinat, Diphenhydramin, Dolasetron, Domperidon, Dronabinol, Droperidol, Granisetron, Haloperidol, Lorazepam, Medizin, Methylprednisolon, Metoclopramid, Ondansetron, Perphenazin, Prochlorperazin, Promethazin, Scopolamin, Tributin, Triethylperazin, Triflupromazin, Trimethobenzamid, Tropisetron, sowie deren Salze, Ester, Hydrate, Polymorphe und Isomere;
- Antiglaukoma, wie Alprenoxim, Dapiprazol, Dipivatefrin, Latanoprost, Naboctat, Pimabin, sowie deren Salze, Ester, Hydrate, Polymorphe und Isomere;
- Antihistaminika, wie Acepromazin, Acrivastin, Activastin, Albuterol, Alimemazin, Antazolin, Azelastin, Bitolterol, Amlexanox, Benzydamin, Brompheniramin, Cetirizin, Chlorpheniramin, Cimetidin, Cinnarizin, Clemastin, Clofedanol, Cycloheptazin, Cyproheptadin, Diclofenac, Difencloxazin, Diphenhydramin, Dotarizin, Ephedrin, Epinastin, Epinephrin, Ethylnorepinephrin, Etybenzatropin, Fenpentadiol, Fenpoterol, Fexofenadin, Flurbiprofen, Hydroxyzin, Isoetharin, Isoproterenol, Ipratropiumbromid, Ketorolac, Levocetirizin, Levomepromazin, Loratidin, Mequitazin, Metaproterenol, Niaprazin, Oxatomid, Oxomemazin, Phenylephrin, Phenylpropanolamin, Pirbuterol, Promethazin, Pseudoephedrin, Pyrilamin, Salmeterol, Terbutalin, Terfenadin, Tranilast, Xanthinderivate, Xylometazolin, sowie deren Salze, Ester, Hydrate, Polymorphe und Isomere;
- Antiinfektiva, wie Antibiotika, Antimykotika, antivirale Mittel wie Abacavir, Acyclovir, Albendazol, Amantadin, Amphotericin, Amikacin, Aminosalicylsäure, Amoxycillin, Ampicillin, Amprenavir, Atovaquine, Azithromycin, Aztreonam, Carbenicillin, Cefaclor, Cefadroxil, Cefamandol, Cefazolin, Cefdinir, Cefepim, Cefexim, Cefoperazone, Cefotaxim, Cefotitam, Cefoperazon, Cefoxitin, Cefpodoxin, Cefprozil, Ceftazidim, Ceftibuten, Ceftizoxim, Ceftriaxon, Cefuroxim, Cephalexin, Chloroquin, Cidofovir, Cilastatin, Ciprofloxacin, Clarithromycin, Clavulansäure, Clindamycin, Colistimethat, Dalfopristin, Dapson, Daunorubicin, Delavirdin, Demeclocyclin, Didanosin, Doxycyclin, Doxorubicin, Efavirenz, Enoxacin, Erythromycin, Ethambutol, Ethionamid, Famcyclovir, Fluconazol, Flucytocin, Foscarnet, Fosfomycin, Ganciclovir, Gatifloxacin, Griseofulvin, Hydroxychloroquin, Imipenem, Indinavir, Interferon, Isoniazid, Itraconazol, Ivermectil, Ketoconazol, Lamivudin, Levofloxacin, Linizolid, Lomefloxacin, Loracarbef, Mebendazol, Mefloquin, Meropenem, Methanamin, Metronidazol, Minocyclin, Moxfloxacin, Naldixinsäure, Nelfinavir, Neomycin, Nevirapin, Nitorfurantoin, Norfloxacin, Ofloxacin, Oseltamivir, Oxytetracyclin, Palivizumab, Penicillin, Perfloxacin, Piperacillin, Praziquantel, Pyrazinamid, Pyrimethamin, Chinidin, Quinupristin, Retonavir, Ribavirin, Rifabutin, Rifampicin, Rimantadin, Saquinavir, Sparfloxacin, Stavudin, Streptomycin, Sulfamethoxazol, Tetramycin, Terbinafin, Tetracyclin, Ticarcillin, Thiabendazol, Tobramycin, Trimethoprim, Trimetraxat, Troleandomycin, Trovafloxacin, Valacyclovir, Vancomycin, Zalcitabin, Zanamivir, Zidovudin, sowie deren Salze, Ester, Hydrate, Polymorphe und Isomere;
- Antiparkinsonmittel, wie Amantadin, Adrogolid, Altiniclin, Benzatropin, Biperiden, Brasofensin, Bromocriptin, Budipin, Cabergolin, CHF-1301, Dihydrexidin, Entacapon, Etilevodopa, Idazoxan, lometopan, Lazabemid, Melevodopa, Carbidopa, Levodopa, Mofegilin, Moxiraprin, Pergolid, Pramipexol, Chineloran, Rasagilin, Ropinirol, Seligilin, Talipexol, Tolcapon, Trihexyphenidyl, sowie deren Salze, Ester, Hydrate, Polymorphe und Isomere;
- Antirheumatika, wie Azathiprin, Betamethason, Celecoxib, Cyclosporin, Diclofenac, Hydroxychloroquin, Indomethacin, Infliximab, Mercaptobutandisäure, Methylprednisolon, Naproxen, Penicillamin, Piroxicam, Prednisolon, Sulfasalazin, sowie deren Salze, Ester, Hydrate, Polymorphe und Isomere;
- Thrombozytenaggregationshemmer, wie Abciximab, Anagrelid, Aspirin, Cilostazol, Clopidogrel, Dipyridamol, Epoprostenol, Eptifibatid, Ticlopidin, Tinofiban, sowie deren Salze, Ester, Hydrate, Polymorphe und Isomere;
- krampflösende und anticholinerge Mittel, wie Aspirin, Atropin, Diclofenac, Hyoscyamin, Mesoprostol, Methocarbamol, Phenobarbital, Scopolamin, sowie deren Salze, Ester, Hydrate, Polymorphe und Isomere;
- Antitussiva, wie Acetaminophen, Acrivastin, Albuterol, Benzonatat, Beractant, Brompheniramin, Koffein, Calfactant, Carbetapentan, Chlorpheniramin, Codein, Colfuscerin, Dextromethorpham, Dornase alpha, Doxylamin, Epinephrin, Fexofenadin, Guaifenesin, Ipratropium, Levalbuterol, Metaproterenol, Montelukast, Pentoxyphillin, Phenylephrin, Phenylpropanolamin, Pirbuterol, Poractant alpha, Pseudoephedrin, Pyrilamin, Salbuterol, Salmeterol, Terbutalin, Theophyllin, Zafirlukast, Zileuton, sowie deren Salze, Ester, Hydrate, Polymorphe und Isomere;
- Carboanhydrasehemmer, wie Acetazolamid, Dichlorphenamid, Dorzolamid, Methazolamid, Sezolamid, sowie deren Salze, Ester, Hydrate, Polymorphe und Isomere;
- Herz-Kreislauf-Mittel, wie Lipidsenker, Antiarrhythmika, Vasodilatatoren, Antianginosa, Antihypertensiva, Vasoprotektiva wie Abciximab, Acebutolol, Aktivase, Adenosin, Adrenalin, Amidaron, Amilorid, Amlodipin, Amylnitrat, Atenolol, Atorvastatin, Benzepril, Bepiridil, Betaxalol, Bisoprolol, Candesartan, Captopril, Cartenolol, Carvedilol, Cerivastatin, Chlorthalidon, Chlorthiazol, Clofibrat, Clonidin, Colestipol, Colesevelam, Digoxin, Diltiazem, Disopyramid, Dobutamin, Dofetilid, Doxazosin, Enalapril, Epoprostenol, Eprosartan, Esmolol, Ethacrynat, Erythrityl, Felodipin, Fenoidapam, Fosinopril, Flecainid, Flurosemid, Fluvastatin, Gemfibrozil, Hydrochlorthiazid, Hydroflumethazin, Ibutilid, Indapamid, Isosorbid, Irbesartan, Labetolol, Lacidipin, Lisinopril, Losartan, Lovastatin, Mecamylamin, Metoprolol, Metarminol, Metazolon, Methylchlothiazid, Methyldopa, Metyrosin, Mexiletin, Midrodin, Milrinon, Moexipril, Nadolol, Niacin, Nicardipin, Nicorandil, Nifedipin, Nimodipin, Nisoldipin, Nitroglycerin, Phenoxybenzamin, Perindopril, Polythiazid, Pravastatin, Prazosin, Procainamid, Propafenon, Propranolol, Quanfacin, Quinapril, Chinidin, Ranipril, Reteplase, Simvastatin, Sotalol, Spironolacton, Streptokinase, Telmisartan, Terazosin, Timolol, Tocainamid, Torsemid, Trandolapril, Triamteren, Trapidil, Valsartan, sowie deren Salze, Ester, Hydrate, Polymorphe und Isomere;
- Vasodilatatoren, wie Adenosin, Alverin, Koffein, Dihydroergocornin, Enalapril, Enoximon, Iloprost, Kalleon, Lidoflazin, Nicardipin, Nimodipin, Nikotinsäure, Papaverin, Pilocarpin, Salbutamol, Theophyllin, Trandolapril, Uradipil, Vincamin, sowie deren Salze, Ester, Hydrate, Polymorphe und Isomere;
- Cholinesterasehemmer, wie Donepezil, Edrophonium, Neostigmin, Pyridostigmin, Rivastigmin, Tacrin, sowie deren Salze, Ester, Hydrate, Polymorphe und Isomere;
- Mittel zur Behandlung von Störungen des zentralen Nervensystems,
- Stimulanzien des zentralen Nervensystems, wie Koffein, Doxapram, Dexoamphetamin, Donepezil, Edorphonium, Methamphetamin, Methylphenidat, Modafinil, Neostigmin, Pemolin, Phentermin, Pyridostigmin, Rivastigmin, Tacrin, sowie deren Salze, Ester, Hydrate, Polymorphe und Isomere;
- Kontrazeptiva, wie Desogestral, Ethinyl-Östradiol, Ethynodiol, Levonorgestrel, Medroxyprogesteron, Mestranol, Norgestimat, Norrethindron, Norgestrel, sowie deren Salze, Ester, Hydrate, Polymorphe und Isomere;
- Fruchtbarkeitsförderer,
- Induktoren und Inhibitoren der Gebärmutterwehen,
- Mittel zur Behandlung von Mukoviszidose, wie Domase alpha, Pankrelipase, Tobramycin, sowie deren Salze, Ester, Hydrate, Polymorphe und Isomere;
- Dopaminrezeptor-Agonisten, wie Amantadin, Cabergolin, Fenoldopam, Pergolid, Pramipezal, Ropinirol, sowie deren Salze, Ester, Hydrate, Polymorphe und Isomere;
- Mittel zur Behandlung von Endometriose, wie Danazol, Goserelin, Leuprolid, Nafarelin, Norethindron, sowie deren Salze, Ester, Hydrate, Polymorphe und Isomere;
- Mittel zur Behandlung der erektilen Dysfunktion, wie Alprostadil, Sildenafil, Yohimbin, sowie deren Salze, Ester, Hydrate, Polymorphe und Isomere;
- Mittel zur Behandlung der Fruchtbarkeit, wie Citrorelix, Clomiphen, Follitropin, Ganirelix, Gonadotropin, Menotropin, Progesteron, Urofollitropin, sowie deren Salze, Ester, Hydrate, Polymorphe und Isomere;
- Mittel zur Behandlung von Magen-Darm-Beschwerden, wie Alosetron, Bisacodyl, Bismutsubsalicylat, Celecoxib, Cimetidin, Difoxin, Dipheoxylat, Docusat, Esomeprazol, Famotidin, Glycopyrrolat, Infliximab, Lansoprazol, Loperamid, Metaclopramid, Nizatidin, Omeprazol, Pantoprazol, Rabeprazol, Ranitidin, Simethicon, Sucralfat, sowie deren Salze, Ester, Hydrate, Polymorphe und Isomere;
- Immunmodulatoren und Immunsuppressiva, wie Azathioprin, Ceftizoxin, Cyclosporin, Daclizumab, Glatiramer, Immunglobulin, Interferon, Leflunomid, Levamisol, Mycophenolat, Phthalidomid, Ribavirin, Sirolimus, sowie deren Salze, Ester, Hydrate, Polymorphe und Isomere;
- Mittel zur Behandlung von Gedächtnisstörungen,
- Migränemittel, wie Acetaminophen, Dihydroergotamin, Divalproex, Ergotamin, Propranolol, Risatriptan, Sumatriptan, Trimetrexat, sowie deren Salze, Ester, Hydrate, Polymorphe und Isomere;
- Muskelrelaxantien, wie Alcuroniumchlorid, Azapropazon, Atracurium, Baclofen, Carisoprodol, Chininderivate, Chlormezanon, Chlorphenesincarbamat, Chlorozoxazon, Cyclobenzaprin, Dantrolen, Decamethoniumbromid, Dimethyltubocurariniumchlorid, Doxacurium, Fenyramidol, Gallamintriethiodid, Guaifenesin, Hexafluoreniumbromid, Hexacarbacholinbromid, Memantin, Mephenesin, Meprobamat, Metamisol, Metaxalon, Methocarbamol, Mivacurium, Orphenadrin, Pancuronium, Phenazon, Phenprobamat, Pipecuronium, Rapacuronium, Rocuronium, Succinylcholin, Suxamethoniumchlorid, Tetrazepam, Tizanidin, Tubocurarinchlorid, Tybamat, Vecuronium, sowie deren Salze, Ester, Hydrate, Polymorphe und Isomere;
- Nukleosidanaloga, wie Abacavir, Acyclovir, Didanosin, Gammiclovir, Gemcitabin, Lamivudin, Ribavirin, Stavudin, Zalcitabin, sowie deren Salze, Ester, Hydrate, Polymorphe und Isomere;
- Mittel zur Behandlung von Osteoporose, wie Alendronat, Calcitonin, Estradiol, Estropipat, Medroxyprogesteron, Norethindron, Norgestimat, Pamidronat, Raloxifen, Risdronat, Zoledronat, sowie deren Salze, Ester, Hydrate, Polymorphe und Isomere;
- Parasympathomimetika, wie Bethanechol, Biperidin, Edrophonium, Glycopyrolat, Hyoscyamin, Pilocarpin, Tacrin, Yohimbin, sowie deren Salze, Ester, Hydrate, Polymorphe und Isomere;
- Prostaglandine, wie Alprostadil, Epoprostenol, Misoprostol, sowie deren Salze, Ester, Hydrate, Polymorphe und Isomere;
- Psychotherapeutische Wirkstoffe, wie Acetophenazin, Alentemol, Alpertin, Alprazolam, Amitriptylin, Apriprazol, Azaperon, Batelapin, Befipirid, Benperidol, Benzindopyrin, Bimithil, Biriperon, Brofoxin, Bromperidol, Broniperidol, Bupropion, Buspiron, Butaclamol, Butaperazin, Carphenazin, Carvotrolin, Cericlamin, Chlorazepin, Chlordiazepoxid, Chlorpromazin, Chlorprothixen, Cinperen, Cintriamid, Citalopram, Clomacran, Clonazepam, Clopenthixol, Clopimozid, Clopipazan, Cloroperon, Clothiapin, Clothixamid, Clozapin, Cyclophenazin, Dapiprazol, Dapoxetin, Desipramin, Divalproex, Dipyridamol, Doxepin, Droperidol, Duloxetin, eltoprazin, Eptipiron, Etazolat, Fenimid, Flibanserin, Flucindol, Flumezapin, Fluoxetin, Fluphenazin, Fluspiperon, Fluspirilen, Flutrolin, Fluvoxamin, Gepiron, Gevotrolin, Halopemid, Haloperidol, Hydroxyzin, Hydroxynortriptylin, Iloperidon, Imidolin, Lamotrigin, Loxapin, Enperon, Mazapertin, Mephobarbital, Meprobamat, Mesoridazin, Mesoridazin, Milnacipran, Mirtazepin, Metiapin, Milenperon, Milipertin, Molindon, Nafadotrid, Naranol, Nefazodon, Neflumozid, Ocaperidon, Odapipam, Olanzapin, Oxethiazin, Oxypromid, Pagoclon, Paliperidon, Paroxiten, Penfluridol, Pentiapin, Perphenazin, Phenelzin, Pimozid, Pinoxepin, Pipamperon, Piperacetazin, Pipotiazin, Piquindon, Pirlindol, Pivagabin, Pramipexol, Prochlorperazin, Promazin, Quetiapin, Reboxetin, Remoxiprid, Risperidon, Rimcazol, Robolzotan, Selegilin, Seperidol, Sertralin, Sertindol, Seteptilin, Setoperon, Spiperon, Sunipitron, Tepirindol, Thioridazin, Thiothixen, Tiaprid, Tioperidon, Tiospiron, Topiramat, Tranylcypromin, Trifluoperazin, Trifluperidol, Triflupromazin, Trimipramin, Venlafaxin, Ziprasidon, sowie deren Salze, Ester, Hydrate, Polymorphe und Isomere;
- Sedativa, Hypnotika und Tranquilizer, wie Bromazepam, Buspiron, Clazolam, Clobazam, Chlorazepat, Diazepam, Demoxepam, Dexmedetomidin, Diphenyhydramin, Doxylamin, Enciprazin, Estrazolam, Hydroxyzin, Ketazolam, Lorazaton, Lorazepam, Loxapin, Medazepam, Meperidin, Methobarbital, Midazolam, Nabilon, Nisobamat, Oxazepam, Pentobarbital, Promethazin, Propofol, Triazolam, Zaleplon, Zolpidem, sowie deren Salze, Ester, Hydrate, Polymorphe und Isomere;
- die Neuroleptika,
- die Anxiolytika,
- die Psychostimulanzien,
- die Antidepressiva,
- Mittel zur dermatologischen Behandlung, wie Acitretin, Alclometason, Alitretinoin, Betamethason, Calciprotrin, Chlorhexidin, Clobetasol, Clocortolon, Clotriamozol, Kollagenase, Cyclosporin, Desonid, Difluoroson, Doxepin, Eflomithin, Finasterid, Fluocinolon, Flurandrenolid, Fluticason, Halobetasol, Hydrochloroquin, Hydrochinon, Hydroxyzin, Ketoconazol, Mafenid, Malathion, Menobenzon, Neostigmin, Nystatin, Podofilox, Povidon, Tazoroten, Tretinoin, sowie deren Salze, Ester, Hydrate, Polymorphe und Isomere;
- Steroide und Hormone, wie Alclometason, Betamethason, Calcitonin, Citrorelix, Clobetasol, Clocortolon, Kortisone, Danazol, Desmopressin, Desonid, Desogestrel, Desoximetason, Dexamethason, Diflorason, Östradiol, Östrogene, Estropipat, Ethynilestradiol, Fluocinolon, Flurandrenolid, Fluticason, Glucagon, Gonadotropin, Goserelin, Halobetasol, Hydrocortison, Leuprolid, Levonorgestrel, Levothyroxin, Medroxyprogesteron, Menotropine, Methylprednisolon, Methyltestosteron, Mometason, Naferelin, Norditropin, Norethindron, Norgestrel, Octreolid, Oxandrolon, Oxymetholon, Polytropin, Prednicarbat, Prednisolon, Progesteron, Sermorelin, Somatropin, Stanozolol, Testosteron, Urofollitropin, sowie deren Salze, Ester, Hydrate, Polymorphe und Isomere.

16. Pharmazeutische, veterinärmedizinische oder diätetische Formulierung, **dadurch gekennzeichnet, dass** sie eine Vielzahl von beschichteten Mikropartikeln, wie in einem der Ansprüche 1 bis 15 definiert, vorteilhafterweise mindestens 500 Mikropartikel, umfasst und dass sie außerdem mindestens einen Wirkstoff in einer Form mit sofortiger Freisetzung umfasst.

17. Formulierung nach Anspruch 16, die eine Vielzahl von Populationen von Mikropartikeln umfasst, wobei sich die Populationen voneinander durch ihre Latenzzeit und/oder durch ihren Auslöse-pH-Wert und/oder durch ihre Freisetzungsgeschwindigkeit und/oder durch den enthaltenen Wirkstoff unterscheiden.

18. Verwendung von beschichteten Mikropartikeln, wie in einem der Ansprüche 1 bis 15 definiert, zur Herstellung von pharmazeutischen, veterinärmedizinischen oder diätetischen Formulierungen, wie in einem der Ansprüche 16 und 17 definiert.

## Claims

1. Coated "reservoir" microparticles containing at least one active principle (AP) and being of the type:
- constituted by AP particles each covered with at least two different coating films (A and B),
- with a mean diameter of less than 2000 microns, **characterized in that**, each of them comprises:
* at least one coating film (A) having the following composition:
- between 10 an 90% by weight of at least one film-forming (co)polymer (A1) that is insoluble in the liquids of the gastrointestinal tract;
- between 5 and 50% by weight of at least one (co)polymer (A2) that is soluble in the liquids of the gastrointestinal tract;
- between 1 and 30% by weight of at least one plasticizer (A3);
- optionally, between 0 and 20% by weight of at least one surfactant and/or lubricant (A4);
* and at least one coating film (B) of the following composition:
- at least one hydrophilic polymer (B1) bearing groups that are ionized at neutral pH,
- at least one hydrophobic compound (B2);
Wherein the weight ratio (B2)/(B1) is comprised between 0,2 and 1,5;
and **in that**, in combination, the coating films (A and B)are capable:
- of ensuring release of the AP governed by two different triggering mechanisms, one based on a variation in pH and the other allowing the release of the AP after a predetermined residence time in the stomach,
- of inducing *in vitro* dissolution behavior (performed in a paddle dissolutest in accordance with the European Pharmacopea 5.2 or a device of type II in US Pharmacopea 28-NF23, maintained at 37°C and rotated at 100 rpm) such that:
- at constant pH 1.4, the dissolution profile comprises a lag phase of adjustable duration of less than or equal to 8 hours;
- the passage from pH 1.4 to pH 7.1 leads to a sustained-release phase of adjustable duration, starting without a lag time and such that t½ is between 0.25 and 20 hours, in which t½ is the time required to release 50% of at least one of the active principles contained in the coated microparticles.

2. Microparticles according to Claim 1, **characterized in that** the mean diameter of said coated microparticles is comprised between 50 and 800 microns.

3. Microparticles according to Claim 2, **characterized in that** the mean diameter of said coated microparticles is comprised between 100 and 600 microns.

4. Microparticles according to Claim 1, in which the coating film A has a proportion of film coating (Tp_{A}) - expressed as a percentage of solids relative to the total mass of the microparticles - such that 2% ≤ Tp_{A} ≤ 50%.

5. Microparticles according to Claim 1, in which the coating film B has a film-coating proportion (Tp_{B}) - expressed as a percentage of solids relative to the total mass of the coated microparticles - of less than or equal to 50%.

6. Microparticles as claimed in any one of claims 1 to 5, in which the coating films A and B together represent not more than 50% of the dry weight relative to the total mass of the coated microparticles.

7. The microparticles as claimed in one of claims 1 to 6, in which:
* (A1) is chosen from among:
• water-insoluble cellulose derivatives,
• acrylic derivatives,
• polyvinyl acetates,
• and mixtures thereof;
* (A2) is chosen from among:
• nitrogenous (co)polymers, selected from the group comprising polyacrylamides, poly-N-vinyl-amides, polyvinylpyrrolidones (PVP) and poly-N-vinyllactams;
• water-soluble cellulose derivatives,
• polyvinyl alcohols (PVA),
• polyoxyethylenes (POE),
• polyethylene glycols (PEG),
• and mixtures thereof;
* (A3) is chosen from among:
• glycerol and esters thereof,
• phthalates,
• citrates,
• sebacates,
• adipates,
• azelates,
• benzoates,
• plant oils,
• fumarates
• oxalates
• succinates
• butyrates,
• cetyl alcohol esters,
• salicylic acid,
• triacetin,
• malonates
• castor oil;
and their mixtures;
* (A4) is chosen from:
• anionic surfactants, selected from the subgroup of alkali metal or alkaline-earth metal salts of fatty acids,
• nonionic surfactants, selected from the following subgroup:
∘ polyoxyethylenated oils,
∘ polyoxyethylene-polyoxypropylene copolymers,
∘ polyoxyethylenated sorbitan esters,
∘ polyoxyethylenated castor oil derivatives,
∘ stearates,
∘ stearylfumarates,
∘ glyceryl behenates,
∘ and mixtures thereof.

8. Microparticles according to Claim 7, in which (A1) is selected from ethylcellulose, cellulose acetate, copolymers of (meth) acrylic acid and of alkyl (e.g. methyl) ester, copolymers of acrylic and methacrylic acid ester bearing at least one quaternary ammonium group and mixtures thereof.

9. Microparticles according to any one of Claims 1 to 8, in which the coating film A has the following quantitative weight percentage composition:
(A1) between 15 and 80,
(A2) between 10 and 40,
(A3) between 2 and 20,
(A4) between 0 and 15.

10. Microparticles as claimed in any one of claims 1 to 9, in which the hydrophilic polymer bearing groups that are ionized at neutral pH (B1) is chosen from:
∘ B1.a copolymers of (meth)acrylic acid and of alkyl (e.g. methyl) ester of (meth)acrylic acid (EUDRAGIT^{®} S or L);
∘ B1.b cellulose derivatives,cellulose phthalates, cellulose succinates;
∘ and mixtures thereof.

11. Microparticles as claimed in any one of claims 1 to 10, in which compound (B2) is chosen from:
∘ B2.a waxes taken alone or as mutual mixtures;
∘ B2.b hydrogenated plant oils taken alone or as mutual mixtures;
∘ B2.c di- and/or triesters of glycerol and of at least one fatty acid;
∘ B2.d and mixtures thereof.

12. Microparticles as claimed in any one of claims 1 to 10, in which compound (B2) is chosen from:
hydrogenated cottonseed oil, hydrogenated soybean oil, hydrogenated palm oil, glyceryl behenate, hydrogenated castor oil, tristearine, tripalmitine, trimyristine, yellow wax, hard fat or fat useful as suppository bases, anhydrous dairy fat, lanolin, glyceryl palmitostearate, glyceryl stearate, lauryl macrogolglycerides, polyglyceryl diisostearate, and any mixture thereof.

13. Microparticles as claimed in Claim 1, in which the weight ratio (B2)/(B1) is comprised 0.45 and 1.0.

14. Microparticles as claimed in Claim 1, in which the hydrophobic compound (B2) is chosen from products that are crystalline in the solid state and that have a melting point Tm(B2) ≥ 40°C.

15. Microparticles as claimed in any one of the preceding claims, in which the AP used belongs to at least one of the following families of active substances:
- agents for treating alcohol abuse such as chlorazepate, chlordiazepoxide, diazepam, disulfiram, hydroxyzine, naltrexone, and salts thereof, esters thereof, hydrates thereof, polymorphs thereof and isomers thereof,
- agents for treating Alzheimer's disease, such as CP 118954, donepezil, galanthamine, metrifonate, revastigmine, tacrine, TAK- 147, and salts thereof, esters thereof, hydrates thereof, polymorphs thereof and isomers thereof;
- anesthetics such as adrenalin, bupivacaine, chloroprocaine, desflurane, etidocaine, levobupivacaine, lidocaine, midazolam, propofol, ropivacaine, and salts thereof, esters thereof, hydrates thereof, polymorphs thereof and isomers thereof,
- agents for treating acromegaly, such as octreotide, laureotide and pegvisomant, and salts thereof, esters thereof, hydrates thereof, polymorphs thereof and isomers thereof;
- analgesics, such as acetaminophen, aspirin, bupivacaine, buprenorphine, butorphanol, celecoxib, clofenadol, choline, clonidine, codeine, diflunisal, dihydrocodeine, dihydroergotamine, dihydromorphine, ethylmorphine, etodolac, eletriptan, eptazocine, ergotamine, fentanyl, fenoprofen, hyaluronic acid, hydrocodone, hydromorphone, hylane, ibuprofen, indomethacin, ketorolac, ketotifen, levomethadone, levallorphan, levorphanol, lidocaine, mefenamic acid, meloxicam, meperidine, methadone, morphine, nabumetone, nalbuphine, nefopam, nalorphine, naloxone, naltrexone, naproxen, naratriptan, nefazodone, mormethadone, oxapozine, oxycodone, oxymorphone, pentazocine, pethidine, phenpyramide, piritramide, piroxicam, propoxyphen, refecoxib, rizatriptan, ketoprofen, sulindac, sumatriptan, tebacone, tilidine, tolmetine, tramadol, zolmitriptan, and salts thereof, esters thereof, hydrates thereof, polymorphs thereof and isomers thereof;
- antiasthmatic agents, such as ablukast, azelastine, bunaprolast, cinalukast, cromitrile, cromolyne, enofelast, isambxole, ketotifen, levcromekaline, lodoxamide, montelukast, ontazolast, oxarbazole, oxatomide, piriprost potassium, pirolate, pobilukast, edamine, pranlukast, quazolast, repirinast, ritolukast, sulukast, tetrazolastmeglumine, tiaramide, tibenelast, tomelukast, tranilast, verlukast, verofylline, zarirlukast, and salts thereof, esters thereof, hydrates thereof, polymorphs thereof and isomers thereof;
- agents for treating allergies,
- anticancer agents, such as adriamycin, aldesleukin, allopurinol, altretamine, amifostine, anastrozole, asparaginase, betamethasone, bexaroten, bicalutamide, bleomycin, busulfan, capecitabine, carboplatin, carmustine, chlorambucil, cisplatin, cladribine, conjugated estrogen, cortisone, cyclophosphamide, cytarabine, dacarbazine, daunorubicin, dactinomycin, denileukin, dexamethasone, discodermolide, docetaxel, doxorubicin, eloposidem, epirubicin, epoetin, epothilones, estramustine, esterified estrogen, ethynyl-estradiol, etoposide, exemestane, flavopirdol, fluconazole, fludarabine, fluorouracil, flutamide, floxuridine, gemcitabine, gemtuzumab, goserelin, hexamethylmelamine, hydrocortisone, hydroxyurea, idarubicin, ifosfamide, interferon, irinotecan, lemiposide, letrozole, leuprolide, levamisole, levothyroxine, lomustine, mechlorethamine, melphalan, mercaptopurine, megestrol, methotrexate, methylprednisolone, methyltestosterone, mithramycin, mitomycin, mitotane, mitoxantrone, mitozolomide, mutamycin, nilutamide, paclitaxel, pamidronate, pegaspargase, pentostatin, plicamycin, porfimer, prednisolone, procarbazine, rituximab, sargramostim, semustine, streptozocin, tamoxifen, temozolamide, teniposide, testolactone, thioguanine, thiotepa, tomudex, topotecan, toremifen, trastumuzab, tretinoin, semustine, streptozolocin, valrubicin, verteprofin, vinblastine, vincristine, vindesine, vinorelbine, and salts thereof, esters thereof, hydrates thereof, polymorphs thereof and isomers thereof;
- antiinflammatories,
- anticoagulants and antithrombotic agents, such as warfarin, dalteparine, heparine, tinzaparin, enoxaparin, danaparoid, abciximab, alprostadil, altiplase, anagralide, anistreplase, argatroban, ataprost, betaprost, camonagrel, cilostazol, clinprost, clopidogrel, cloricromen, dermatan, desirudine, domitroban, drotaverine, epoprostenol, eptifibatide, fradafiban, gabexate, iloprost, isbogrel, lamifiban, lamoteplase, lefradafiban, lepirudin, levosimendan, lexipafant, melagatran, nafagrel, nafamostsat, nizofenone, orbifiban, ozagrel, pamicogrel, parnaparin, quinobendan, reteplase, sarpogralate, satigrel, silteplase, simendan, ticlopidine, vapiprost, tirofiban, xemilofiban, Y20811, and salts thereof, esters thereof, hydrates thereof, polymorphs thereof and isomers thereof;
- antiepileptic agents,
- antidiabetic agents such as acarbose, acetohexamide, carbutamide, chlorpropamide, epalrestat, glibornuride, gliclazide, glimepiride, glipizide, gliquidone, glisoxepide, glyburide, glyhexamide, metformin, miglitol, nateglinide, orlistat, phenbutamide, pioglitazone, repaglinide, rosiglitazone, tolazamide, tolbutamide, tolcyclamide, tolrestat, troglitazone, voglibose, and salts thereof, esters thereof, hydrates thereof, polymorphs thereof and isomers thereof;
- antiemetic agents, such as alprazolam, benzquinamide, benztropine, betahistine, chlorpromazine, dexamethasone, difenidol, dimenhydrinate, diphenhydramine, dolasetron, domperidone, dronabinol, droperidol, granisetron, haloperidol, lorazepam, meclizine, methylprednisolone, metoclopramide, ondansetron, perphenazine, prochlorperazine, promethazine, scopolamine, tributin, triethylperazine, triflupromazine, trimethobenzamide, tropisetron, and salts thereof, esters thereof, hydrates thereof, polymorphs thereof and isomers thereof;
- antiglaucoma agents, such as alprenoxime, dapiprazole, dipivefrin, latanoprost, naboctate, pimabine, and salts thereof, esters thereof, hydrates thereof, polymorphs thereof and isomers thereof;
- antihistamines such as acepromazine, acrivastine, activastine, albuterol, alimemazine, antazoline, azelastine, bitolterol, amlexanox, benzydamine, brompheniramine, cetirizine, chlorpheniramine, cimetidine, cinnarizine, clemastine, clofedanol, cycloheptazine, cyproheptadine, diclofenac, difencloxazine, diphenhydramine, dotarizine, ephedrine, epinastine, epinephrine, ethylnorepinephrine, etybenzatropine, fenpentadiol, fenpoterol, fexofenadine, flurbiprofen, hydroxyzine, isoetharine, isoproterenol, ipratropium bromide, ketorolac, levocetirizine, levomepromazine, loratidine, mequitazine, metaproterenol, niaprazine, oxatomide, oxomemazine, phenylephrine, phenylpropanolamine, pirbuterol, promethazine, pseudoephedrine, pyrilamine, salmeterol, terbutaline, terfenadine, tranilast, xanthine derivatives, xylometazoline, and salts thereof, esters thereof, hydrates thereof, polymorphs thereof and isomers thereof;
- antiinfectious agents such as antibiotics, antifungal agents, antiviral agents, such as abacavir, acyclovir, albendazole, amantadine, amphotericin, amikacin, aminosalicylic acid, amoxycillin, ampicillin, amprenavir, atovaquine, azithromycin, aztreonam, carbenicillin, cefaclor, cefadroxil, cefamandole, cefazolin, cefdinir, cefepime, cefexime, cefoperazone, cefotaxime, cefotitam, cefoperazone, cefoxitine, cefpodoxine, cefprozil, ceftazidime, ceftibuten, ceftizoxime, ceftriaxone, cefuroxime, cephalexine, chloroquine, cidofovir, cilastatin, ciprofloxacin, clarithromycin, clavulanic acid, clindamycin, colistimethate, dalfopristin, dapsone, daunorubicin, delavirdine, demeclocycline, didanosine, doxycycline, doxorubicin, efavirenz, enoxacin, erythromycin, ethambutol, ethionamide, famcyclovir, fluconazole, flucytocine, foscarnet, fosfomycin, ganciclovir, gatifloxacin, griseofulvin, hydroxychloroquine, imipenem, indinavir, interferon, isoniazide, itraconazole, ivermectil, ketoconazole, lamivudin, levofloxacin, linizolide, lomefloxacin, loracarbef, mebendazole, mefloquine, meropenem, methanamine, metronidazole, minocycline, moxefloxacin, naldixic acid, nelfinavir, neomycin, nevirapine, nitorfurantoin, norfloxacin, ofloxacin, oseltamivir, oxytetracycline, palivizumab, penicillin, perfloxacin, piperacillin, praziquantel, pyrazinamide, pyrimethamine, quinidine, quinupristine, retonavir, ribavirin, rifabutin, rifampicin, rimantadine, saquinavir, sparfloxacin, stavudin, streptomycin, sulfamethoxazole, tetramycin, terbinafine, tetracycline, ticarcillin, thiabendazole, tobramycin, trimethoprim, trimetraxate, troleandomycin, trovafloxacin, valacyclovir, vancomycin, zalcitabine, zanamivir, zidovudine, and salts thereof, esters thereof, hydrates thereof, polymorphs thereof and isomers thereof;
- antiparkinson agents such as amantadine, adrogolide, altinicline, benzatropine, biperiden, brasofensine, bromocriptine, budipine, cabergoline, CHF-1301, dihydrexidine, entacapone, etilevodopa, idazoxane, iometopane, lazabemide, melevodopa, carbidopa, levodopa, mofegiline, moxiraprine, pergolide, pramipexole, quinelorane, rasagiline, ropinirole, seligiline, talipexole, tolcapone, trihexyphenidyl, and salts thereof, esters thereof, hydrates thereof, polymorphs thereof and isomers thereof;
- antirheumatic agents such as azathiprine, betamethasone, celecoxib, cyclosporine, diclofenac, hydroxychloroquine, indomethacin, infliximab, mercaptobutanedioic acid, methylprednisolone, naproxen, penicillamine, piroxicam, prednisolone, sulfasalazine, and salts thereof, esters thereof, hydrates thereof, polymorphs thereof and isomers thereof;
- anti-platelet-aggregating agents such as abciximab, anagrelide, aspirin, cilostazol, clopidogrel, dipyridamole, epoprostenol, eptifibatide, ticlopidine, tinofiban, and salts thereof, esters thereof, hydrates thereof, polymorphs thereof and isomers thereof;
- antispasmodic and anticholinergic agents such as aspirin, atropine, diclofenac, hyoscyamine, mesoprostol, methocarbamol, phenobarbital, scopolamine, and salts thereof, esters thereof, hydrates thereof, polymorphs thereof and isomers thereof;
- antitussive agents such as acetaminophen, acrivastine, albuterol, benzonatate, beractant, brompheniramine, caffeine, calfactant, carbetapentane, chlorpheniramine, codeine, colfuscerine, dextromethorpham, dornase alpha, doxylamine, epinephrine, fexofenadine, guaphenesin, ipratropium, levalbuterol, metaproterenol, montelukast, pentoxyphilline, phenylephrine, phenylpropanolamine, pirbuterol, poractant alpha, pseudoephedrine, pyrilamine, salbuterol, salmeterol, terbutaline, theophylline, zafirlukast, zileuton, and salts thereof, esters thereof, hydrates thereof, polymorphs thereof and isomers thereof;
- carbonic anhydrase inhibitors include: acetazolamide, dichlorphenamide, dorzolamide, methazolamide, sezolamide, and salts thereof, esters thereof, hydrates thereof, polymorphs thereof and isomers thereof;
- cardiovascular agents, such as hypolipemiants, antiarrhythmic agents, vasodilators, antiangina agents, antihypertensives and vasoprotective agents, such as abciximab, acebutolol, activase, adenosine, adrenaline, amidarone, amiloride, amlodipine, amyl nitrate, atenolol, atorvastatin, benzepril, bepiridil, betaxalol, bisoprolol, candesartan, captopril, cartenolol, carvedilol, cerivastatin, chlorthalidone, chlorthiazole, clofibrate, clonidine, colestipol, colosevelam, digoxin, diltiazem, disopyramide, dobutamine, dofetilide, doxazosine, enalapril, epoprostenol, eprosartan, esmolol, ethacrynate, erythrityl, felodipine, fenoidapam, fosinopril, flecainide, flurosemide, fluvastatin, gemfibrozil, hydrochlorthiazide, hydroflumethazine, ibutilide, indapamide, isosorbide, irbesartan, labetolol, lacidipine, lisinopril, losartan, lovastatin, mecamylamine, metoprolol, metarminol, metazolone, methylchlothiazide, methyldopa, metyrosine, mexiletine, midrodine, milrinone, moexipril, nadolol, niacin, nicardipine, nicorandil, nifedipine, nimodipine, nisoldipine, nitroglycerine, phenoxybenzamine, perindopril, polythiazide, pravastatin, prazosin, procainamide, propafenone, propranolol, quanfacine, quinapril, quinidine, ranipril, reteplase, simvastatin, sotalol, spironolactone, streptokinase, telmisartan, terazosin, timolol, tocainamide, torsemide, trandolapril, triamteren, trapidil, valsartan, and salts thereof, esters thereof, hydrates thereof, polymorphs thereof and isomers thereof;
- vasodilators such as adenosine, alverine, caffeine, dihydroergocornine, enalapril, enoximone, iloprost, kalleone, lidoflazine, nicardipine, nimodipine, nicotinic acid, papaverine, pilocarpine, salbutamol, theophylline, trandolapril, uradipil, vincamine, and salts thereof, esters thereof, hydrates thereof, polymorphs thereof and isomers thereof;
- cholinesterase inhibitors such as donepezil, edrophonium, neostigmine, pyridostigmine, rivastigmine, tacrine, and salts thereof, esters thereof, hydrates thereof, polymorphs thereof and isomers thereof;
- agents for treating central nervous system disorders,
- central nervous system stimulants such as caffeine, doxapram, dexoamphetamine, donepezil, edorphonium, methamphetamine, methylphenidate, modafinil, neostigmine, pemoline, phentermine, pyridostigmine, rivastigmine, tacrine, and salts thereof, esters thereof, hydrates thereof, polymorphs thereof and isomers thereof;
- contraceptives such as desogestral, ethinylestradiol, ethynodiol, levonorgestrel, medroxyprogesterone, mestranol, norgestimate, norethindrone, norgestrel, and salts thereof, esters thereof, hydrates thereof, polymorphs thereof and isomers thereof;
- fertility promoters,
- labor inducers and inhibitors,
- agents for treating mucoviscidosis such as domase alpha, pancrelipase, tobramycin, and salts thereof, esters thereof, hydrates thereof, polymorphs thereof and isomers thereof;
- dopamine receptor agonists such as amantadine, cabergoline, fenoldopam, pergolide, pramipezal, ropinirole, and salts thereof, esters thereof, hydrates thereof, polymorphs thereof and isomers thereof;
- agents for treating endometriosis include: danazol, goserelin, leuprolide, nafarelin, norethindrone, and salts thereof, esters thereof, hydrates thereof, polymorphs thereof and isomers thereof;
- agents for treating erectile dysfunction such as alprostadil, sildenafil, yohimbine, and salts thereof, esters thereof, hydrates thereof, polymorphs thereof and isomers thereof;
- agents for treating fertility such as citrorelix, clomiphen, follitropine, ganirelix, gonadotropin, menotropin, progesterone, urofollitropin, and salts thereof, esters thereof, hydrates thereof, polymorphs thereof and isomers thereof;
- agents for treating gastrointestinal disorder such as alosetron, bisacodyl, bismuth subsalicylate, celecoxib, cimetidine, difoxine, dipheoxylate, docusate, esomeprazole, famotidine, glycopyrrolate, infliximab, lansoprazole, loperamide, metaclopramide, nizatidine, omeprazole, pantoprazole, rabeprazole, ranitidine, simethicone, sucralfate, and salts thereof, esters thereof, hydrates thereof, polymorphs thereof and isomers thereof;
- immunomodulators and immunosuppressants include: azathioprine, ceftizoxine, cyclosporine, daclizumab, glatiramer, immunoglobulin, interferon, leflunomide, levamisol, mycophenolate, phthalidomide, ribavirine, sirolimus, and salts thereof, esters thereof, hydrates thereof, polymorphs thereof and isomers thereof;
- agents for treating memory disorders,
- antimigraine agents such as acetaminophen, dihydroergotamine, divalproex, ergotamine, propranolol, risatriptan, sumatriptan, trimetrexate, and salts thereof, esters thereof, hydrates thereof, polymorphs thereof and isomers thereof;
- muscle relaxants such as alcuronium chloride, azapropazone, atracurium, baclofen, carisoprodol, quinine derivatives, chloromezanone, chlorophenesincarbamate, chlorozoxazone, cyclobenzaprine, dantrolen, decamethonium bromide, dimethyltubocurarinium chloride, doxacurium, fenyramidol, gallamine triethiodide, guaiphenesin, hexafluorenium bromide, hexacarbacholine bromide, memantin, mephenesin, meprobamate, metamisol, metaxalone, methocarbamol, mivacurium, orphenadrine, pancuronium, phenazone, phenprobamate, pipecuronium, rapacuronium, rocuronium, succinylcholine, suxamethonium chloride, tetrazepam, tizanidine, tubocurarine chloride, tybamate, vecuronium, and salts thereof, esters thereof, hydrates thereof, polymorphs thereof and isomers thereof;
- nucleoside analogs such as abacavir, acyclovir, didanosine, gamciclovir, gemcitabine, lamivudine, ribavirin, stavudine, zalcitabine, and salts thereof, esters thereof, hydrates thereof, polymorphs thereof and isomers thereof;
- agents for treating osteoporosis such as alendronate, calcitonin, estradiol, estropipate, medroxyprogesterone, norethindrone, norgestimate, pamidronate, raloxifen, risdronate, zoledronate, and salts thereof, esters thereof, hydrates thereof, polymorphs thereof and isomers thereof;
- parasympathomimetic agents such as bethanechol, biperidine, edrophonium, glycopyrolate, hyoscyamine, pilocarpine, tacrine, yohimbine, and salts thereof, esters thereof, hydrates thereof, polymorphs thereof and isomers thereof;
- prostaglandins such as alprostadil, epoprostenol, misoprostol, and salts thereof, esters thereof, hydrates thereof, polymorphs thereof and isomers thereof;
- psychotherapeutic agents such as acetophenazine, alentemol, alpertine, alprazolam, amitriptyline, apriprazole, azaperone, batelapine, befipiride, benperidol, benzindopyrine, bimithil, biriperone, brofoxine, bromperidol, broniperidol, bupropione, buspirone, butaclamol, butaperazine, butaperazin, carphenazine, carvotroline, cericlamine, chlorazepine, chlordiazepoxide, chlorpromazine, chlorprothixen, cinperen, cintriamide, citalopram, clomacran, clonazepam, clopenthixol, clopimozide, clopipazan, cloroperone, clothiapine, clothixamide, clozapine, cyclophenazine, dapiprazole, dapoxetine, desipramine, divalproex, dipyridamole, doxepin, droperidol, duloxetine, eltoprazine, eptipirone, etazolate, fenimide, flibanserine, flucindole, flumezapine, fluoxetine, fluphenazine, fluspiperone, fluspirilen, flutroline, fluvoxamine, gepirone, gevotroline, halopemide, haloperidol, hydroxyzine, hydroxynortriptyline, iloperidone, imidoline, lamotrigine, loxapine, enperone, mazapertine, mephobarbital, meprobamate, mesoridazine, mesoridazine, milnacipran, mirtazepine, metiapine, milenperone, milipertine, molindone, nafadotride, naranol, nefazodone, neflumozide, ocaperidone, odapipam, olanzapine, oxethiazine, oxiperomide, pagoclone, paliperidone, paroxiten, penfluridol, pentiapine, perphenazine, phenelzine, pimozide, pinoxepin, pipamperone, piperacetazine, pipotiazine, piquindone, pirlindole, pivagabine, pramipexole, prochlorperazine, promazine, quetiapine, reboxetine, remoxipride, risperidone, rimcazole, robolzotan, selegiline, seperidol, sertraline, sertindole, seteptiline, setoperone, spiperone, sunipitrone, tepirindole, thioridazine, thiothixen, tiapride, tioperidone, tiospirone, topiramate, tranylcypromine, trifluoperazine, trifluperidol, triflupromazine, trimipramine, venlafaxine, ziprasidone, and salts thereof, esters thereof, hydrates thereof, polymorphs thereof and isomers thereof;
- sedatives, hypnotics and tranquilizers such as bromazepam, buspirone, clazolam, clobazam, chlorazepate, diazepam, demoxepam, dexmedetomidine, diphenyhydramine, doxylamine, enciprazine, estrazolam, hydroxyzine, ketazolam, lorazatone, lorazepam, loxapine, medazepam, meperidine, methobarbital, midazolam, nabilone, nisobamate, oxazepam, pentobarbital, promethazine, propofol, triazolam, zaleplon, zolpidem, and salts thereof, esters thereof, hydrates thereof, polymorphs thereof and isomers thereof;
- neuroleptics, anxyolitics, psychostimulants, antidepressants,
- dermatological treatment agents such as acitretin, alclometasone, alitretinoin, betamethasone, calciprotrine, chlorhexidine, clobetasol, clocortolone, clotriamozole, collagenase, cyclosporine, desonide, difluorosone, doxepine, eflomithine, finasteride, fluocinolone, flurandrenolide, fluticasone, halobetasol, hydrochloroquine, hydroquinone, hydroxyzine, ketoconazole, mafenide, malathion, menobenzone, neostigmine, nystatin, podofilox, povidone, tazoroten, tretinoin, and salts thereof, esters thereof, hydrates thereof, polymorphs thereof and isomers thereof;
- steroids and hormones such as alclometasone, betamethasone, calcitonine, citrorelix, clobetasol, clocortolone, cortisones, danazol, desmopressin, desonide, desogestrel, desoximetasone, dexamethasone, diflorasone, estradiol, estrogens, estropipate, ethynilestradiol, fluocinolone, flurandrenolide, fluticasone, glucagon, gonadotropin, goserelin, halobetasol, hydrocortisone, leuprolide, levonorgestrel, levothyroxine, medroxyprogesterone, menotropins, methylprednisolone, methyltestosterone, mometasone, naferelin, norditropin, norethindrone, norgestrel, octreolide, oxandrolone, oxymetholone, polytropin, prednicarbate, prednisolone, progesterone, sermorelin, somatropin, stanozolol, testosterone, urofollitropin, and salts thereof, esters thereof, hydrates thereof, polymorphs thereof and isomers thereof.

16. A pharmaceutical, veterinary or dietetic formulation, **characterized in that** it comprises a plurality of coated microparticles as claimed in any one of claims 1 to 15, advantageously at least 500, and **in that** it further comprises at least an active principle in an immediate release form.

17. A formulation according to Claim 16, **characterized in that** it comprises a plurality of populations of microparticles, said populations differing from each other by their lag time and/or by their triggering pH and/or by their rate of release and/or by the active principle they contain.

18. The use of the coated microparticles as claimed in any one of claims 1 to 15, for the preparation of pharmaceutical, veterinary or dietetic formulations as defined in any one of claims 16 and 17.
